# EUROPEAN PATENT SPECIFICATION

(11) **EP 4 114 975 B1**
(45) Date of publication and mention of the grant of the patent: **29.04.2026**
(21) Application number: 21715058.0
(22) Date of filing: 05.03.2021
(51) Int. Cl.: C07K 14/47

(54) **FASCIN-2 (FSCN2) VARIANT AND USE THEREOF**
FASCIN-2 (FSCN2) VARIANTE UND DEREN VERWENDUNG
VARIANT DE LA FASCINE-2 (FSCN2) ET LEUR UTILISATION

(30) Priority: 06.03.2020 US 202062985947 P; 04.10.2020 US 202063087249 P
(43) Date of publication of application: 11.01.2023
(73) Proprietor: Regeneron Pharmaceuticals, Inc., Tarrytown, NY 10591 (US)
(72) Inventor: PRAVEEN, Kavita, Tarrytown, New York 10591 (US); COPPOLA, Giovanni, Tarrytown, New York 10591 (US); FERREIRA, Manuel Allen Revez, Tarrytown, New York 10591 (US); GURSKI, Lauren, Tarrytown, New York 10591 (US); BARAS, Aris, Tarrytown, New York 10591 (US); DRUMMOND SAMUELSON, Meghan, Tarrytown, New York 10591 (US); ABECASIS, Goncalo, Tarrytown, New York 10591 (US)
(74) Representative: Vossius & Partner Patentanwälte Rechtsanwälte mbB
(86) International application number: PCT/US2021/021219
(87) International publication number: WO 2021/178904

(56) References cited:
- WO-A1-2009/094713
- LIU XIANG ET AL: "Null Mutation of the Fascin2 Gene by TALEN Leading to Progressive Hearing Loss and Retinal Degeneration in C57BL/6J Mice", G3 GENES|GENOMES|GENETICS, vol. 8, no. 10, 1 October 2018 (2018-10-01), pages 3221 - 3230, XP055808233, Retrieved from the Internet <URL:https://www.ncbi.nlm.nih.gov/pmc/articles/PMC6169377/pdf/3221.pdf> DOI: 10.1534/g3.118.200405
- SHIN J.-B. ET AL: "The R109H Variant of Fascin-2, a Developmentally Regulated Actin Crosslinker in Hair-Cell Stereocilia, Underlies Early-Onset Hearing Loss of DBA/2J Mice", THE JOURNAL OF NEUROSCIENCE, vol. 30, no. 29, 21 July 2010 (2010-07-21), US, pages 9683 - 9694, XP055808238, ISSN: 0270-6474, Retrieved from the Internet <URL:https://www.ncbi.nlm.nih.gov/pmc/articles/PMC2922854/pdf/zns9683.pdf> DOI: 10.1523/JNEUROSCI.1541-10.2010
- HILGERT NELE ET AL: "Forty-six genes causing nonsyndromic hearing impairment: Which ones should be analyzed in DNA diagnostics?", MUTATION RESEARCH. REVIEWS IN MUTATION RESEARCH, ELSEVIER, AMSTERDAM, NL, vol. 681, no. 2, 29 August 2008 (2008-08-29), pages 189 - 196, XP086402953, ISSN: 1383-5742, [retrieved on 20080829], DOI: 10.1016/J.MRREV.2008.08.002
- MB PETERSEN ET AL: "Non-syndromic, autosomal-recessive deafness : Autosomal-recessive deafness", CLINICAL GENETICS, vol. 69, no. 5, 27 April 2006 (2006-04-27), DK, pages 371 - 392, XP055713939, ISSN: 0009-9163, DOI: 10.1111/j.1399-0004.2006.00613.x

## Description

### Field

The present disclosure relates generally to the treatment of subjects having hearing loss, methods of identifying subjects having an increased risk of developing hearing loss, and methods of detecting FSCN2 variant nucleic acid molecules and variant polypeptides.

### Background

Hearing impairment is the most common sensory defect in humans, affecting approximately 15% of Americans. Studies have shown that approximately 1 in 1000 infants are born with a level of deafness that will affect their linguistic development and speech perception (McHugh and Friedman, Anat. Rec. A Discov. Mol. Cell Evol. Biol., 2006, 288, 370-381). Hearing loss usually has a negative impact on education, social interactions and career opportunities and is, therefore, associated with major social and financial costs. The impact of hearing loss upon an individual can be quite significant (Yoshinaga-Itano, Otolaryngol Clin. North Am., 1999, 32, 1089-1102; Mohr and Feldman, Int. J. Technol. Assess. Health Care, 2000, 16, 1120-35).

The degree of hearing loss varies from mild to profound deafness. It can be stable or the hearing loss can progress with time. The deafness can be present at birth or develop late in life. By far the largest group of people with hearing loss are amongst the elderly, many of whom develop an age-related hearing loss or presbyacusis (Petit, Trends Mol. Med., 2006, 12, 57-64). Both genetic and environmental factors can cause deafness. Genetic factors are the underlying aetiology of deafness in the majority of children and young people with hearing loss. Genetic factors play a significant role in age-related hearing loss. Hearing loss can be associated with other clinical features (syndromic hearing loss) or isolated (non-syndromic hearing loss) (Petersen and Willems, Clin. Genet., 2006, 69, 37I-392).

FSCN2 is a member of the fascin protein family. Fascins crosslink actin into filamentous bundles within dynamic cell extensions. FSCN2 is proposed to play a role in photoreceptor disk morphogenesis. A mutation in this gene results in one form of autosomal dominant retinitis pigmentosa and macular degeneration. FCSN2 appears to play a role in organizing a special subset of arrays of actin filaments comprising membrane-enveloped protrusions of tightly bundled parallel actin filaments, which include filopodia, microvilli, and inner ear stereocilia. These protrusions enhance the ability of cells to transduce extracellular signals and transport essential metabolites. Stereocilia of the inner ear's sensory hair cells are particularly exaggerated examples of actin protrusions. Dozens to hundreds of coupled stereocilia make up the hair bundle, the organelle in the inner ear that transduces sound or head movements into electrical signals. FSCN2 has been reported to be abundant in hair cells and is developmentally regulated, appearing in inner-hair-cell stereocilia during final stages of elongation. Late expression and genetic interaction with cadherin 23 suggest that FSCN2 plays a critical role in stabilizing stereocilia after development. Liu Xiang et al., Genes, 8, 10, pp.3221 (2018); Shin et al., J. Neuroscinces, 30, pp. 9683 (2010) discusses the role of the FSCN2 gene and ear function.

### Summary

The present invention is defined in the appended claims. The present disclosure provides methods of identifying a subject having an increased risk for developing hearing loss, wherein the methods comprise: determining or having determined the presence or absence of an FSCN2 predicted loss-of-function variant nucleic acid molecule encoding a human FSCN2 polypeptide in a biological sample obtained from the subject; wherein: when the subject is FSCN2 reference, then the subject does not have an increased risk for developing hearing loss; and when the subject is heterozygous for an FSCN2 predicted loss-of-function variant or homozygous for an FSCN2 predicted loss-of-function variant, then the subject has an increased risk for developing hearing loss.

The present disclosure also provides methods of treating a subject with a therapeutic agent that treats or inhibits hearing loss, wherein the subject is suffering from hearing loss, the methods comprising the steps of: determining whether the subject has an FSCN2 predicted loss-of-function variant nucleic acid molecule encoding a human FSCN2 polypeptide by: obtaining or having obtained a biological sample from the subject; and performing or having performed a sequence analysis on the biological sample to determine if the subject has a genotype comprising the FSCN2 predicted loss-of-function variant nucleic acid molecule; and when the subject is FSCN2 reference, then administering or continuing to administer to the subject the therapeutic agent that treats or inhibits hearing loss in a standard dosage amount; and when the subject is heterozygous or homozygous for an FSCN2 predicted loss-of-function variant, then administering or continuing to administer to the subject the therapeutic agent that treats or inhibits hearing loss in an amount that is the same as or greater than a standard dosage amount; wherein the presence of a genotype having the FSCN2 predicted loss-of-function variant nucleic acid molecule encoding the human FSCN2 polypeptide indicates the subject has an increased risk of developing hearing loss.

The present disclosure also provides methods of detecting a human FSCN2 variant nucleic acid molecule in a subject comprising assaying a sample obtained from the subject to determine whether a nucleic acid molecule in the sample is: i) a genomic nucleic acid molecule comprising a nucleotide sequence comprising a thymine at a position corresponding to position 548 according to SEQ ID NO:2, or the complement thereof; ii) an mRNA molecule comprising a nucleotide sequence comprising: a uracil at a position corresponding to position 548 according to SEQ ID NO:6, or the complement thereof; a uracil at a position corresponding to position 469 according to SEQ ID NO:7, or the complement thereof; a uracil at a position corresponding to position 553 according to SEQ ID NO:8, or the complement thereof; or a uracil at a position corresponding to position 567 according to SEQ ID NO:20, or the complement thereof; or iii) a cDNA molecule comprising a nucleotide sequence comprising: a thymine at a position corresponding to position 548 according to SEQ ID NO:12, or the complement thereof; a thymine at a position corresponding to position 469 according to SEQ ID NO:13, or the complement thereof; a thymine at a position corresponding to position 553 according to SEQ ID NO:14, or the complement thereof; or a thymine at a position corresponding to position 567 according to SEQ ID NO:22, or the complement thereof.

The present disclosure also provides methods of detecting the presence of a human FSCN2 variant polypeptide, comprising performing an assay on a sample obtained from a subject to determine whether an FSCN2 protein in the sample comprises a tyrosine at a position corresponding to position 138 according to SEQ ID NO:17, or a tyrosine at a position corresponding to position 138 according to SEQ ID NO:18.

The present disclosure also provides therapeutic agents that treat or inhibit hearing loss for use in the treatment of hearing loss in a subject having: i) a genomic nucleic acid molecule having a nucleotide sequence encoding a human FSCN2 polypeptide, wherein the nucleotide sequence comprises a thymine at a position corresponding to position 548 according to SEQ ID NO:2, or the complement thereof; ii) an mRNA molecule having a nucleotide sequence encoding a human FSCN2 polypeptide, wherein the nucleotide sequence comprises: a uracil at a position corresponding to position 548 according to SEQ ID NO:6, or the complement thereof; a uracil at a position corresponding to position 469 according to SEQ ID NO:7, or the complement thereof; a uracil at a position corresponding to position 553 according to SEQ ID NO:8, or the complement thereof; or a uracil at a position corresponding to position 567 according to SEQ ID NO:20, or the complement thereof; or iii) a cDNA molecule having a nucleotide sequence encoding a human FSCN2 polypeptide, wherein the nucleotide sequence comprises: a thymine at a position corresponding to position 548 according to SEQ ID NO:12, or the complement thereof; a thymine at a position corresponding to position 469 according to SEQ ID NO:13, or the complement thereof; a thymine at a position corresponding to position 553 according to SEQ ID NO:14, or the complement thereof; or a thymine at a position corresponding to position 567 according to SEQ ID NO:22, or the complement thereof.

### Brief Description Of The Drawings

Figure 1 shows an association of a missense variant (His138Tyr) in *FSCN2* with increased risk for hearing loss.
Figure 2 shows an association with an aggregate of loss of function and missense variants (minor allele frequency less than 1%) in *FSCN2* suggesting that several variants in *FSCN2* may increase the risk for hearing loss.

### Description

Various terms relating to aspects of the present disclosure are used throughout the specification and claims. Such terms are to be given their ordinary meaning in the art, unless otherwise indicated. Other specifically defined terms are to be construed in a manner consistent with the definitions provided herein.

Unless otherwise expressly stated, it is in no way intended that any method or aspect set forth herein be construed as requiring that its steps be performed in a specific order. Accordingly, where a method claim does not specifically state in the claims or descriptions that the steps are to be limited to a specific order, it is in no way intended that an order be inferred, in any respect. This holds for any possible non-expressed basis for interpretation, including matters of logic with respect to arrangement of steps or operational flow, plain meaning derived from grammatical organization or punctuation, or the number or type of aspects described in the specification.

As used herein, the singular forms "a," "an" and "the" include plural referents unless the context clearly dictates otherwise.

As used herein, the term "about" means that the recited numerical value is approximate and small variations would not significantly affect the practice of the disclosed embodiments. Where a numerical value is used, unless indicated otherwise by the context, the term "about" means the numerical value can vary by ±10% and remain within the scope of the disclosed embodiments.

As used herein, the term "comprising" may be replaced with "consisting" or "consisting essentially of" in particular embodiments as desired.

As used herein, the term "isolated", in regard to a nucleic acid molecule or a polypeptide, means that the nucleic acid molecule or polypeptide is in a condition other than its native environment, such as apart from blood and/or animal tissue. In some embodiments, an isolated nucleic acid molecule or polypeptide is substantially free of other nucleic acid molecules or other polypeptides, particularly other nucleic acid molecules or polypeptides of animal origin. In some embodiments, the nucleic acid molecule or polypeptide can be in a highly purified form, i.e., greater than 95% pure or greater than 99% pure. When used in this context, the term "isolated" does not exclude the presence of the same nucleic acid molecule or polypeptide in alternative physical forms, such as dimers or alternatively phosphorylated or derivatized forms.

As used herein, the terms "nucleic acid", "nucleic acid molecule", "nucleic acid sequence", "polynucleotide", or "oligonucleotide" can comprise a polymeric form of nucleotides of any length, can comprise DNA and/or RNA, and can be single-stranded, doublestranded, or multiple stranded. One strand of a nucleic acid also refers to its complement.

As used herein, the term "subject" is any animal, including mammals. Mammals include, but are not limited to, farm animals (such as, for example, horse, cow, pig), companion animals (such as, for example, dog, cat), laboratory animals (such as, for example, mouse, rat, rabbits), and non-human primates. In some embodiments, the subject is a human.

A rare variant in the FSCN2 gene associated with an increased risk of developing hearing loss, such as conductive hearing loss and/or sensorineural hearing loss, in subjects has been identified in accordance with the present disclosure. For example, a genetic alteration in a cDNA molecule resulting in the replacement of a histidine with a tyrosine at position 138 in the encoded FSCN2 polypeptide has been observed to indicate that the human having such an alteration may have an increased risk of developing hearing loss, such as conductive hearing loss and/or sensorineural hearing loss. Altogether, the genetic analyses described herein surprisingly indicate that the FSCN2 His138Tyr polypeptide associates with an increased risk of developing hearing loss, such as conductive hearing loss and/or neural hearing loss. Therefore, subjects that have an FSCN2 variant nucleic acid molecule or polypeptide that associates with an increased risk of developing hearing loss, such as conductive hearing loss, sensorineural hearing loss, or neural hearing loss, may be treated such that hearing loss is prevented, the symptoms thereof are reduced, and/or development of symptoms is repressed. Accordingly, the present disclosure provides methods of leveraging the identification of such variants in subjects to identify or stratify risk in such subjects of developing hearing loss, such as conductive hearing loss, sensorineural hearing loss, or neural hearing loss, or to diagnose subjects as having an increased risk of developing hearing loss, such as conductive hearing loss, sensorineural hearing loss, or neural hearing loss, such that subjects at risk or subjects with active disease may be treated accordingly.

For purposes of the present disclosure, any particular human can be categorized as having one of three FSCN2 genotypes: i) FSCN2 reference; ii) heterozygous for an FSCN2 predicted loss-of-function variant; or iii) homozygous for an FSCN2 predicted loss-of-function variant. A human is FSCN2 reference when the human does not have a copy of an FSCN2 predicted loss-of-function variant nucleic acid molecule. A human is heterozygous for an FSCN2 predicted loss-of-function variant when the human has a single copy of an FSCN2 predicted loss-of-function variant nucleic acid molecule. AN FSCN2 predicted loss-of-function variant nucleic acid molecule is any FSCN2 nucleic acid molecule (such as, a genomic nucleic acid molecule, an mRNA molecule, or a cDNA molecule) encoding an FSCN2 polypeptide having a partial loss-of-function, a complete loss-of-function, a predicted partial loss-of-function, or a predicted complete loss-of-function. A human who has an FSCN2 polypeptide having a partial loss-of-function (or predicted partial loss-of-function) is hypomorphic for FSCN2. The FSCN2 predicted loss-of-function variant nucleic acid molecule can be any nucleic acid molecule encoding an FSCN2 H138Y polypeptide. In some embodiments, the FSCN2 predicted loss-of-function variant nucleic acid molecule encodes FSCN2 H138Y Long or FSCN2 H138Y Short. A human is homozygous for an FSCN2 predicted loss-of-function variant when the human has two copies of an FSCN2 predicted loss-of-function variant nucleic acid molecule.

For subjects that are genotyped or determined to be heterozygous or homozygous for an FSCN2 predicted loss-of-function variant nucleic acid molecule, such subjects have an increased risk of developing hearing loss, such as conductive hearing loss, sensorineural hearing loss, or neural hearing loss. For subjects that are genotyped or determined to be heterozygous or homozygous for an FSCN2 predicted loss-of-function variant nucleic acid molecule, such subjects can be treated with an agent effective to treat a hearing loss, such as conductive hearing loss, sensorineural hearing loss, or neural hearing loss.

In any of the embodiments described herein, the FSCN2 predicted loss-of-function variant nucleic acid molecule can be any FSCN2 nucleic acid molecule (such as, for example, genomic nucleic acid molecule, mRNA molecule, or cDNA molecule) encoding an FSCN2 polypeptide having a partial loss-of-function, a complete loss-of-function, a predicted partial loss-of-function, or a predicted complete loss-of-function. For example, the FSCN2 predicted loss-of-function variant nucleic acid molecule can be any nucleic acid molecule encoding an FSCN2 H138Y polypeptide. In some embodiments, the FSCN2 predicted loss-of-function variant nucleic acid molecule encodes FSCN2 H138Y Long or FSCN2 H138Y Short.

In any of the embodiments described herein, the FSCN2 predicted loss-of-function polypeptide can be any FSCN2 polypeptide having a partial loss-of-function, a complete loss-of-function, a predicted partial loss-of-function, or a predicted complete loss-of-function. In any of the embodiments described herein, the FSCN2 predicted loss-of-function polypeptide can be any of the FSCN2 polypeptides described herein including, for example, FSCN2 H138Y. In some embodiments, the FSCN2 predicted loss-of-function polypeptide is FSCN2 H138Y Long or FSCN2 H138Y Short.

In any of the embodiments described herein, hearing loss is conductive hearing loss, sensorineural hearing loss, or neural hearing loss. In any of the embodiments described herein, hearing loss is conductive hearing loss. In any of the embodiments described herein, hearing loss is sensorineural hearing loss. In any of the embodiments described herein, hearing loss is neural hearing loss.

Symptoms of hearing loss include, but are not limited to, hearing problem (muffling of speech and other sounds, difficulty understanding words, especially against background noise or in a crowd, or trouble hearing consonants), ringing in the ears, sensitivity to sound, or speech delay in a child.

The present disclosure provides methods of treating a subject with a therapeutic agent that treats or inhibits hearing loss, wherein the subject is suffering from hearing loss. In some embodiments, the methods comprise determining whether the subject has an FSCN2 predicted loss-of-function variant nucleic acid molecule encoding a human FSCN2 polypeptide by obtaining or having obtained a biological sample from the subject, and performing or having performed a sequence analysis on the biological sample to determine if the subject has a genotype comprising the FSCN2 predicted loss-of-function variant nucleic acid molecule. When the subject is FSCN2 reference, the therapeutic agent that treats or inhibits hearing loss is administered or continued to be administered to the subject in a standard dosage amount. When the subject is heterozygous or homozygous for an FSCN2 predicted loss-of-function variant, the therapeutic agent that treats or inhibits hearing loss is administered or continued to be administered to the subject in an amount that is the same as or greater than a standard dosage amount. The presence of a genotype having the FSCN2 predicted loss-of-function variant nucleic acid molecule encoding the human FSCN2 polypeptide indicates the subject has an increased risk of developing hearing loss. In some embodiments, the subject is FSCN2 reference. In some embodiments, the subject is heterozygous for an FSCN2 predicted loss-of-function variant. In some embodiments, the subject is homozygous for an FSCN2 predicted loss-of-function variant.

In some embodiments, the methods of treatment further comprise detecting the presence or absence of an FSCN2 predicted loss-of-function variant nucleic acid molecule encoding a human FSCN2 polypeptide in a biological sample from the subject. As used throughout the present disclosure, a "FSCN2 predicted loss-of-function variant nucleic acid molecule" is any FSCN2 nucleic acid molecule (such as, for example, genomic nucleic acid molecule, mRNA molecule, or cDNA molecule) encoding an FSCN2 polypeptide having a partial loss-of-function, a complete loss-of-function, a predicted partial loss-of-function, or a predicted complete loss-of-function.

Detecting the presence or absence of an FSCN2 predicted loss-of-function variant nucleic acid molecule in a biological sample from a subject and/or determining whether a subject has an FSCN2 predicted loss-of-function variant nucleic acid molecule can be carried out by any of the methods described herein. In some embodiments, these methods can be carried out *in vitro.* In some embodiments, these methods can be carried out *in situ.* In some embodiments, these methods can be carried out *in vivo.* In any of these embodiments, the nucleic acid molecule can be present within a cell obtained from the subject.

The present disclosure also provides methods of treating a subject with a therapeutic agent that treats or inhibits hearing loss, wherein the subject is suffering from hearing loss. In some embodiments, the method comprises determining whether the subject has an FSCN2 predicted loss-of-function polypeptide by obtaining or having obtained a biological sample from the subject, and performing or having performed an assay on the biological sample to determine if the subject has an FSCN2 predicted loss-of-function polypeptide. When the subject does not have an FSCN2 predicted loss-of-function polypeptide, the therapeutic agent that treats or inhibits hearing loss is administered or continued to be administered to the subject in a standard dosage amount. When the subject has an FSCN2 predicted loss-of-function polypeptide, the therapeutic agent that treats or inhibits hearing loss is administered or continued to be administered to the subject in an amount that is the same as or greater than a standard dosage amount. The presence of an FSCN2 predicted loss-of-function polypeptide indicates the subject has an increased risk of developing hearing loss. In some embodiments, the subject has an FSCN2 predicted loss-of-function polypeptide. In some embodiments, the subject does not have an FSCN2 predicted loss-of-function polypeptide.

Detecting the presence or absence of an FSCN2 predicted loss-of-function polypeptide in a biological sample from a subject and/or determining whether a subject has an FSCN2 predicted loss-of-function polypeptide can be carried out by any of the methods described herein. In some embodiments, these methods can be carried out *in vitro.* In some embodiments, these methods can be carried out *in situ.* In some embodiments, these methods can be carried out *in vivo.* In any of these embodiments, the polypeptide can be present within a cell obtained from the subject.

Examples of therapeutic agents that treat or inhibit hearing loss include, but are not limited to: antioxidants, calcium-channel blockers, anti-inflammatory drugs (such as steroids), apoptosis inhibitors, D-methionine, ebselen, N-acetylcysteine, lipoic acid, combination of ebselen and allopurinol, resveratrol, neurotrophic factors (such as T-817MA), caspase inhibitors (such as z-DEVD-fmk), copper transport inhibitors (such as cimetidine and copper sulphate), and micronutrients with antioxidant vitamins.

In some embodiments, the dose of the therapeutic agents that treat or inhibit hearing loss can be increased by about 10%, by about 20%, by about 30%, by about 40%, by about 50%, by about 60%, by about 70%, by about 80%, or by about 90% for subjects that are heterozygous or homozygous for an FSCN2 predicted loss-of-function variant (i.e., a greater amount than the standard dosage amount) compared to subjects that are FSCN2 reference (who may receive a standard dosage amount). In some embodiments, the dose of the therapeutic agents that treat or inhibit hearing loss can be increased by about 10%, by about 20%, by about 30%, by about 40%, or by about 50%. In addition, the dose of therapeutic agents that treat or inhibit hearing loss in subjects that are heterozygous or homozygous for an FSCN2 predicted loss-of-function variant can be administered more frequently compared to subjects that are FSCN2 reference.

In some embodiments, the dose of the therapeutic agents that treat or inhibit hearing loss can be increased by about 10%, by about 20%, by about 30%, by about 40%, by about 50%, by about 60%, by about 70%, by about 80%, or by about 90% for subjects that are homozygous for an FSCN2 predicted loss-of-function variant compared to subjects that are heterozygous for an FSCN2 predicted loss-of-function variant. In some embodiments, the dose of the therapeutic agents that treat or inhibit hearing loss can be increased by about 10%, by about 20%, by about 30%, by about 40%, or by about 50%. In addition, the dose of therapeutic agents that treat or inhibit hearing loss in subjects that are homozygous for an FSCN2 predicted loss-of-function variant can be administered more frequently compared to subjects that are heterozygous for an FSCN2 predicted loss-of-function variant.

Administration of the therapeutic agents that treat or inhibit hearing loss can be repeated, for example, after one day, two days, three days, five days, one week, two weeks, three weeks, one month, five weeks, six weeks, seven weeks, eight weeks, two months, or three months. The repeated administration can be at the same dose or at a different dose. The administration can be repeated once, twice, three times, four times, five times, six times, seven times, eight times, nine times, ten times, or more. For example, according to certain dosage regimens a subject can receive therapy for a prolonged period of time such as, for example, 6 months, 1 year, or more.

Administration of the therapeutic agents that treat or inhibit hearing loss can occur by any suitable route including, but not limited to, parenteral, intravenous, oral, subcutaneous, intra-arterial, intracranial, intrathecal, intraperitoneal, topical, intranasal, or intramuscular. Pharmaceutical compositions for administration are desirably sterile and substantially isotonic and manufactured under GMP conditions. Pharmaceutical compositions can be provided in unit dosage form (i.e., the dosage for a single administration). Pharmaceutical compositions can be formulated using one or more physiologically and pharmaceutically acceptable carriers, diluents, excipients or auxiliaries. The formulation depends on the route of administration chosen. The term "pharmaceutically acceptable" means that the carrier, diluent, excipient, or auxiliary is compatible with the other ingredients of the formulation and not substantially deleterious to the recipient thereof.

The terms "treat", "treating", and "treatment" and "prevent", "preventing", and "prevention" as used herein, refer to eliciting the desired biological response, such as a therapeutic and prophylactic effect, respectively. In some embodiments, a therapeutic effect comprises one or more of a decrease/reduction in hearing loss, a decrease/reduction in the severity of hearing loss (such as, for example, a reduction or inhibition of development or hearing loss), a decrease/reduction in symptoms and hearing loss-related effects, delaying the onset of symptoms and hearing loss-related effects, reducing the severity of symptoms of hearing loss-related effects, reducing the severity of an acute episode, reducing the number of symptoms and hearing loss-related effects, reducing the latency of symptoms and hearing loss-related effects, an amelioration of symptoms and hearing loss-related effects, reducing secondary symptoms, reducing secondary infections, preventing relapse to hearing loss, decreasing the number or frequency of relapse episodes, increasing latency between symptomatic episodes, increasing time to sustained progression, speeding recovery, and/or increasing efficacy of or decreasing resistance to alternative therapeutics, following administration of the agent or composition comprising the agent. A prophylactic effect may comprise a complete or partial avoidance/inhibition or a delay of hearing loss development/progression (such as, for example, a complete or partial avoidance/inhibition or a delay), following administration of a therapeutic protocol. Treatment of hearing loss encompasses the treatment of subjects already diagnosed as having any form of hearing loss at any clinical stage or manifestation, the delay of the onset or evolution or aggravation or deterioration of the symptoms or signs of hearing loss, and/or preventing and/or reducing the severity of hearing loss.

The present disclosure also provides methods of identifying a subject having an increased risk for developing hearing loss. In some embodiments, the method comprises determining or having determined in a biological sample obtained from the subject the presence or absence of an FSCN2 predicted loss-of-function variant nucleic acid molecule (such as a genomic nucleic acid molecule, mRNA molecule, and/or cDNA molecule) encoding a human FSCN2 polypeptide. When the subject lacks an FSCN2 predicted loss-of-function variant nucleic acid molecule (i.e., the subject is genotypically categorized as an FSCN2 reference), then the subject does not have an increased risk for developing hearing loss. When the subject has an FSCN2 predicted loss-of-function variant nucleic acid molecule (i.e., the subject is heterozygous for an FSCN2 predicted loss-of-function variant or homozygous for an FSCN2 predicted loss-of-function variant), then the subject has an increased risk for developing hearing loss.

Determining whether a subject has an FSCN2 predicted loss-of-function variant nucleic acid molecule in a biological sample from a subject and/or determining whether a subject has an FSCN2 predicted loss-of-function variant nucleic acid molecule can be carried out by any of the methods described herein. In some embodiments, these methods can be carried out *in vitro.* In some embodiments, these methods can be carried out *in situ.* In some embodiments, these methods can be carried out *in vivo.* In any of these embodiments, the nucleic acid molecule can be present within a cell obtained from the subject.

In some embodiments, when a subject is identified as having an increased risk of developing hearing loss, the subject is further treated with a therapeutic agent that treats or inhibits hearing loss, as described herein. In some embodiments, when the subject is heterozygous or homozygous for an FSCN2 predicted loss-of-function variant, the subject is administered the therapeutic agent that treats or inhibits hearing loss in a dosage amount that is the same as or greater than a standard dosage amount. In some embodiments, when the subject is homozygous for an FSCN2 predicted loss-of-function variant, the subject is administered the therapeutic agent that treats or inhibits hearing loss in a dosage amount that is the same as or greater than the dosage amount administered to a subject that is heterozygous for an FSCN2 predicted loss-of-function variant. In some embodiments, the subject is FSCN2 reference. In some embodiments, the subject is heterozygous for an FSCN2 predicted loss-of-function variant. In some embodiments, the subject is homozygous for an FSCN2 predicted loss-of-function variant.

The present disclosure also provides methods of detecting the presence or absence of an FSCN2 predicted loss-of-function variant genomic nucleic acid molecule in a biological sample from a subject, and/or an FSCN2 predicted loss-of-function variant mRNA molecule in a biological sample from a subject, and/or an FSCN2 predicted loss-of-function variant cDNA molecule produced from an mRNA molecule in a biological sample from a subject. It is understood that gene sequences within a population and mRNA molecules encoded by such genes can vary due to polymorphisms such as single-nucleotide polymorphisms. The sequences provided herein for the FSCN2 variant genomic nucleic acid molecule, FSCN2 variant mRNA molecule, and FSCN2 variant cDNA molecule are only exemplary sequences. Other sequences for the FSCN2 variant genomic nucleic acid molecule, variant mRNA molecule, and variant cDNA molecule are also possible.

The biological sample can be derived from any cell, tissue, or biological fluid from the subject. The sample may comprise any clinically relevant tissue, such as a bone marrow sample, a tumor biopsy, a fine needle aspirate, or a sample of bodily fluid, such as blood, gingival crevicular fluid, plasma, serum, lymph, ascitic fluid, cystic fluid, or urine. In some cases, the sample comprises a buccal swab. The sample used in the methods disclosed herein will vary based on the assay format, nature of the detection method, and the tissues, cells, or extracts that are used as the sample. A biological sample can be processed differently depending on the assay being employed. For example, when detecting any FSCN2 variant nucleic acid molecule, preliminary processing designed to isolate or enrich the sample for the genomic DNA can be employed. A variety of techniques may be used for this purpose. When detecting the level of any FSCN2 variant mRNA, different techniques can be used enrich the biological sample with mRNA. Various methods to detect the presence or level of an mRNA or the presence of a particular variant genomic DNA locus can be used.

In some embodiments, detecting a human FSCN2 predicted loss-of-function variant nucleic acid molecule in a subject comprises assaying or analyzing a biological sample obtained from the subject to determine whether an FSCN2 genomic nucleic acid molecule in the biological sample, and/or an FSCN2 mRNA molecule in the biological sample, and/or an FSCN2 cDNA molecule produced from an mRNA moleucle in the biological sample, comprises one or more variations that cause a loss-of-function (partial or complete) or are predicted to cause a loss-of-function (partial or complete).

In some embodiments, the methods of detecting the presence or absence of an FSCN2 predicted loss-of-function variant nucleic acid molecule (such as, for example, a genomic nucleic acid molecule, an mRNA molecule, and/or a cDNA molecule produced from an mRNA molecule) in a subject, comprise performing an assay on a biological sample obtained from the subject. The assay determines whether a nucleic acid molecule in the biological sample comprises a particular nucleotide sequence.

In some embodiments, the nucleotide sequence comprises: a thymine at a position corresponding to position 548 according to SEQ ID NO:2 (for genomic nucleic acid molecules), a uracil at a position corresponding to position 548 according to SEQ ID NO:6 (for mRNA molecules), or a thymine at a position corresponding to position 548 according to SEQ ID NO:12 (for cDNA molecules obtained from mRNA molecules).

In some embodiments, the nucleotide sequence comprises a uracil at a position corresponding to position 469 according to SEQ ID NO:7 (for mRNA molecules) or a thymine at a position corresponding to position 469 according to SEQ ID NO:13 (for cDNA molecules obtained from mRNA molecules).

In some embodiments, the nucleotide sequence comprises a uracil at a position corresponding to position 553 according to SEQ ID NO:8 (for mRNA molecules) or a thymine at a position corresponding to position 553 according to SEQ ID NO:14 (for cDNA molecules obtained from mRNA molecules).

In some embodiments, the nucleotide sequence comprises a uracil at a position corresponding to position 567 according to SEQ ID NO:20 (for mRNA molecules) or a thymine at a position corresponding to position 567 according to SEQ ID NO:22 (for cDNA molecules obtained from mRNA molecules).

In some embodiments, the nucleotide sequence comprises a thymine at a position corresponding to position 548 according to SEQ ID NO:2, or the complement thereof.

In some embodiments, the nucleotide sequence comprises: a uracil at a position corresponding to position 548 according to SEQ ID NO:6, or the complement thereof; a uracil at a position corresponding to position 469 according to SEQ ID NO:7, or the complement thereof; a uracil at a position corresponding to position 553 according to SEQ ID NO:8, or the complement thereof; or a uracil at a position corresponding to position 567 according to SEQ ID NO:20, or the complement thereof.

In some embodiments, the nucleotide sequence comprises: a thymine at a position corresponding to position 548 according to SEQ ID NO:12, or the complement thereof; a thymine at a position corresponding to position 469 according to SEQ ID NO:13, or the complement thereof; a thymine at a position corresponding to position 553 according to SEQ ID NO:14, or the complement thereof; or a thymine at a position corresponding to position 567 according to SEQ ID NO:22, or the complement thereof.

In some embodiments, the biological sample comprises a cell or cell lysate. Such methods can further comprise, for example, obtaining a biological sample from the subject comprising an FSCN2 genomic nucleic acid molecule or mRNA molecule, and if mRNA, optionally reverse transcribing the mRNA into cDNA. Such assays can comprise, for example determining the identity of these positions of the particular FSCN2 nucleic acid molecule. In some embodiments, the method is an *in vitro* method.

In some embodiments, the determining step, detecting step, or sequence analysis comprises sequencing at least a portion of the nucleotide sequence of the FSCN2 genomic nucleic acid molecule, the FSCN2 mRNA molecule, or the FSCN2 cDNA molecule in the biological sample, wherein the sequenced portion comprises one or more variations that cause a loss-of-function (partial or complete) or are predicted to cause a loss-of-function (partial or complete).

In some embodiments, the determining step, detecting step, or sequence analysis comprises sequencing at least a portion of: the nucleotide sequence of the FSCN2 genomic nucleic acid molecule in the biological sample, wherein the sequenced portion comprises a position corresponding to position 548 according to SEQ ID NO:2, or the complement thereof; the nucleotide sequence of the FSCN2 mRNA molecule in the biological sample, wherein the sequenced portion comprises a position corresponding to position 548 according to SEQ ID NO:6, or the complement thereof; and/or the nucleotide sequence of the FSCN2 cDNA molecule produced from the mRNA in the biological sample, wherein the sequenced portion comprises a position corresponding to position 548 according to SEQ ID NO:12, or the complement thereof. When the sequenced portion of the FSCN2 nucleic acid molecule in the biological sample comprises: a thymine at a position corresponding to position 548 according to SEQ ID NO:2, a uracil at a position corresponding to position 548 according to SEQ ID NO:6, or a thymine at a position corresponding to position 548 according to SEQ ID NO:12, then the FSCN2 nucleic acid molecule in the biological sample is an FSCN2 predicted loss-of-function variant nucleic acid molecule.

In some embodiments, the determining step, detecting step, or sequence analysis comprises sequencing at least a portion of: the nucleotide sequence of the FSCN2 mRNA molecule in the biological sample, wherein the sequenced portion comprises a position corresponding to position 469 according to SEQ ID NO:7, or the complement thereof; and/or the nucleotide sequence of the FSCN2 cDNA molecule produced from the mRNA in the biological sample, wherein the sequenced portion comprises a position corresponding to position 469 according to SEQ ID NO:13, or the complement thereof. When the sequenced portion of the FSCN2 nucleic acid molecule in the biological sample comprises a uracil at a position corresponding to position 469 according to SEQ ID NO:7, or a thymine at a position corresponding to position 469 according to SEQ ID NO:13, then the FSCN2 nucleic acid molecule in the biological sample is an FSCN2 predicted loss-of-function variant nucleic acid molecule.

In some embodiments, the determining step, detecting step, or sequence analysis comprises sequencing at least a portion of: the nucleotide sequence of the FSCN2 mRNA molecule in the biological sample, wherein the sequenced portion comprises a position corresponding to position 553 according to SEQ ID NO:8, or the complement thereof; and/or the nucleotide sequence of the FSCN2 cDNA molecule produced from the mRNA in the biological sample, wherein the sequenced portion comprises a position corresponding to position 553 according to SEQ ID NO:14, or the complement thereof. When the sequenced portion of the FSCN2 nucleic acid molecule in the biological sample comprises a uracil at a position corresponding to position 553 according to SEQ ID NO:8, or a thymine at a position corresponding to position 553 according to SEQ ID NO:14, then the FSCN2 nucleic acid molecule in the biological sample is an FSCN2 predicted loss-of-function variant nucleic acid molecule.

In some embodiments, the determining step, detecting step, or sequence analysis comprises sequencing at least a portion of: the nucleotide sequence of the FSCN2 mRNA molecule in the biological sample, wherein the sequenced portion comprises a position corresponding to position 567 according to SEQ ID NO:20, or the complement thereof; and/or the nucleotide sequence of the FSCN2 cDNA molecule produced from the mRNA in the biological sample, wherein the sequenced portion comprises a position corresponding to position 567 according to SEQ ID NO:22, or the complement thereof. When the sequenced portion of the FSCN2 nucleic acid molecule in the biological sample comprises a uracil at a position corresponding to position 567 according to SEQ ID NO:20, or a thymine at a position corresponding to position 567 according to SEQ ID NO:22, then the FSCN2 nucleic acid molecule in the biological sample is an FSCN2 predicted loss-of-function variant nucleic acid molecule.

In some embodiments, the determining step, detecting step, or sequence analysis comprises sequencing at least a portion of the nucleotide sequence of the FSCN2 genomic nucleic acid molecule in the biological sample, wherein the sequenced portion comprises a position corresponding to position 548 according to SEQ ID NO:2, or the complement thereof. When the sequenced portion of the FSCN2 nucleic acid molecule in the biological sample comprises a thymine at a position corresponding to position 548 according to SEQ ID NO:2, then the FSCN2 nucleic acid molecule in the biological sample is an FSCN2 predicted loss-of-function variant nucleic acid molecule.

In some embodiments, the determining step, detecting step, or sequence analysis comprises sequencing at least a portion of the nucleotide sequence of the FSCN2 mRNA molecule in the biological sample, wherein the sequenced portion comprises a position corresponding to: position 548 according to SEQ ID NO:6, or the complement thereof; position 469 according to SEQ ID NO:7, or the complement thereof; position 553 according to SEQ ID NO:8, or the complement thereof; or position 567 according to SEQ ID NO:20, or the complement thereof. When the sequenced portion of the FSCN2 nucleic acid molecule in the biological sample comprises: a uracil at a position corresponding to position 548 according to SEQ ID NO:6; a uracil at a position corresponding to position 469 according to SEQ ID NO:7; a uracil at a position corresponding to position 553 according to SEQ ID NO:8; or a uracil at a position corresponding to position 567 according to SEQ ID NO:20; then the FSCN2 nucleic acid molecule in the biological sample is an FSCN2 predicted loss-of-function variant nucleic acid molecule.

In some embodiments, the determining step, detecting step, or sequence analysis comprises sequencing at least a portion of the nucleotide sequence of the FSCN2 cDNA molecule, wherein the sequenced portion comprises a position corresponding to: position 548 according to SEQ ID NO:12, or the complement thereof; position 469 according to SEQ ID NO:13, or the complement thereof; position 553 according to SEQ ID NO:14, or the complement thereof; or position 567 according to SEQ ID NO:22, or the complement thereof. When the sequenced portion of the FSCN2 nucleic acid molecule in the biological sample comprises: a thymine at a position corresponding to position 548 according to SEQ ID NO:12; a thymine at a position corresponding to position 469 according to SEQ ID NO:13; a thymine at a position corresponding to position 553 according to SEQ ID NO:14; or a thymine at a position corresponding to position 567 according to SEQ ID NO:22; then the FSCN2 nucleic acid molecule in the biological sample is an FSCN2 predicted loss-of-function variant nucleic acid molecule.

In some embodiments, the determining step, detecting step, or sequence analysis comprises: a) contacting the biological sample with a primer hybridizing to a portion of the nucleotide sequence of the FSCN2: genomic nucleic acid molecule that is proximate to a position corresponding to position 548 according to SEQ ID NO:2; mRNA molecule that is proximate to a position corresponding to position 548 according to SEQ ID NO:6; and/or cDNA molecule that is proximate to a position corresponding to position 548 according to SEQ ID NO:12; b) extending the primer at least through the position of the nucleotide sequence of the FSCN2: genomic nucleic acid molecule corresponding to position 548 according to SEQ ID NO:2, mRNA molecule corresponding to position 548 according to SEQ ID NO:6, and/or cDNA molecule corresponding to position 548 according to SEQ ID NO:12; and c) determining whether the extension product of the primer comprises: a thymine at a position corresponding to position 548 according to SEQ ID NO:2, a uracil at a position corresponding to position 548 according to SEQ ID NO:6, and/or a thymine at a position corresponding to position 548 according to SEQ ID NO:12.

In some embodiments, the determining step, detecting step, or sequence analysis comprises: a) contacting the biological sample with a primer hybridizing to a portion of the nucleotide sequence of the FSCN2: mRNA molecule that is proximate to a position corresponding to position 469 according to SEQ ID NO:7, and/or cDNA molecule that is proximate to a position corresponding to position 469 according to SEQ ID NO:13; b) extending the primer at least through the position of the nucleotide sequence of the FSCN2: mRNA molecule corresponding to position 469 according to SEQ ID NO:7, and/or cDNA molecule corresponding to position 469 according to SEQ ID NO:13; and c) determining whether the extension product of the primer comprises: a uracil at a position corresponding to position 469 according to SEQ ID NO:7, and/or a thymine at a position corresponding to position 469 according to SEQ ID NO:13.

In some embodiments, the determining step, detecting step, or sequence analysis comprises: a) contacting the biological sample with a primer hybridizing to a portion of the nucleotide sequence of the FSCN2: mRNA molecule that is proximate to a position corresponding to position 553 according to SEQ ID NO:8, and/or cDNA molecule that is proximate to a position corresponding to position 553 according to SEQ ID NO:14; b) extending the primer at least through the position of the nucleotide sequence of the FSCN2: mRNA molecule corresponding to position 553 according to SEQ ID NO:8, and/or cDNA molecule corresponding to position 553 according to SEQ ID NO:14; and c) determining whether the extension product of the primer comprises: a uracil at a position corresponding to position 553 according to SEQ ID NO:8, and/or a thymine at a position corresponding to position 553 according to SEQ ID NO:14.

In some embodiments, the determining step, detecting step, or sequence analysis comprises: a) contacting the biological sample with a primer hybridizing to a portion of the nucleotide sequence of the FSCN2: mRNA molecule that is proximate to a position corresponding to position 567 according to SEQ ID NO:20, and/or cDNA molecule that is proximate to a position corresponding to position 567 according to SEQ ID NO:22; b) extending the primer at least through the position of the nucleotide sequence of the FSCN2: mRNA molecule corresponding to position 567 according to SEQ ID NO:20, and/or cDNA molecule corresponding to position 567 according to SEQ ID NO:22; and c) determining whether the extension product of the primer comprises: a uracil at a position corresponding to position 567 according to SEQ ID NO:20, and/or a thymine at a position corresponding to position 567 according to SEQ ID NO:22.

In some embodiments, the determining step, detecting step, or sequence analysis comprises: a) contacting the biological sample with a primer hybridizing to a portion of the nucleotide sequence of the FSCN2 genomic nucleic acid molecule that is proximate to a position corresponding to position 548 according to SEQ ID NO:2; b) extending the primer at least through the position of the nucleotide sequence of the FSCN2 genomic nucleic acid moleculecorresponding to position 548 according to SEQ ID NO:2; and c) determining whether the extension product of the primer comprises a thymine at a position corresponding to position 548 according to SEQ ID NO:2.

In some embodiments, the determining step, detecting step, or sequence analysis comprises: a) contacting the biological sample with a primer hybridizing to a portion of the nucleotide sequence of the FSCN2 mRNA molecule that is proximate to a position corresponding to: position 548 according to SEQ ID NO:6, position 469 according to SEQ ID NO:7, position 553 according to SEQ ID NO:8, or position 567 according to SEQ ID NO:20; b) extending the primer at least through the position of the nucleotide sequence of the FSCN2 mRNA molecule corresponding to: position 548 according to SEQ ID NO:6, position 469 according to SEQ ID NO:7, position 553 according to SEQ ID NO:8, or position 567 according to SEQ ID NO:20; and c) determining whether the extension product of the primer comprises: a uracil at a position corresponding to position 548 according to SEQ ID NO:6, a uracil at a position corresponding to position 469 according to SEQ ID NO:7, a uracil at a position corresponding to position 553 according to SEQ ID NO:8, or a uracil at a position corresponding to position 567 according to SEQ ID NO:20.

In some embodiments, the determining step, detecting step, or sequence analysis comprises: a) contacting the biological sample with a primer hybridizing to a portion of the nucleotide sequence of the FSCN2 cDNA molecule that is proximate to a position corresponding to: position 548 according to SEQ ID NO:12, position 469 according to SEQ ID NO:13, position 553 according to SEQ ID NO:14, or position 567 according to SEQ ID NO:22; b) extending the primer at least through the position of the nucleotide sequence of the FSCN2 cDNA molecule corresponding to: position 548 according to SEQ ID NO:12, position 469 according to SEQ ID NO:13, position 553 according to SEQ ID NO:14, or position 567 according to SEQ ID NO:22; and c) determining whether the extension product of the primer comprises: a thymine at a position corresponding to position 548 according to SEQ ID NO:12, a thymine at a position corresponding to position 469 according to SEQ ID NO:13, a thymine at a position corresponding to position 553 according to SEQ ID NO:14, or a thymine at a position corresponding to position 567 according to SEQ ID NO:22.

In some embodiments, the assay comprises sequencing the entire nucleic acid molecule. In some embodiments, only an FSCN2 genomic nucleic acid molecule is analyzed. In some embodiments, only an FSCN2 mRNA is analyzed. In some embodiments, only an FSCN2 cDNA obtained from FSCN2 mRNA is analyzed.

In some embodiments, the determining step, detecting step, or sequence analysis comprises: a) amplifying at least a portion of the nucleic acid molecule that encodes the human FSCN2 polypeptide, wherein the amplified portion comprises: a thymine at a position corresponding to position 548 according to SEQ ID NO:2, or the complement thereof; a uracil at a position corresponding to position 548 according to SEQ ID NO:6, or the complement thereof; and/or a thymine at a position corresponding to position 548 according to SEQ ID NO:12, or the complement thereof; b) labeling the amplified nucleic acid molecule with a detectable label; c) contacting the labeled nucleic acid molecule with a support comprising an alteration-specific probe, wherein the alteration-specific probe comprises a nucleotide sequence which hybridizes under stringent conditions to the nucleic acid sequence of the amplified nucleic acid molecule comprising: a thymine at a position corresponding to position 548 according to SEQ ID NO:2, or the complement thereof; a uracil at a position corresponding to position 548 according to SEQ ID NO:6, or the complement thereof; and/or a thymine at a position corresponding to position 548 according to SEQ ID NO:12, or the complement thereof; and d) detecting the detectable label.

In some embodiments, the determining step, detecting step, or sequence analysis comprises: a) amplifying at least a portion of the nucleic acid molecule that encodes the human FSCN2 polypeptide, wherein the amplified portion comprises: a uracil at a position corresponding to position 469 according to SEQ ID NO:7, or the complement thereof; and/or a thymine at a position corresponding to position 469 according to SEQ ID NO:13, or the complement thereof; b) labeling the amplified nucleic acid molecule with a detectable label; c) contacting the labeled nucleic acid molecule with a support comprising an alteration-specific probe, wherein the alteration-specific probe comprises a nucleotide sequence which hybridizes under stringent conditions to the nucleic acid sequence of the amplified nucleic acid molecule comprising: a uracil at a position corresponding to position 469 according to SEQ ID NO:7, or the complement thereof; and/or a thymine at a position corresponding to position 469 according to SEQ ID NO:13, or the complement thereof; and d) detecting the detectable label.

In some embodiments, the determining step, detecting step, or sequence analysis comprises: a) amplifying at least a portion of the nucleic acid molecule that encodes the human FSCN2 polypeptide, wherein the amplified portion comprises: a uracil at a position corresponding to position 553 according to SEQ ID NO:8, or the complement thereof; and/or a thymine at a position corresponding to position 553 according to SEQ ID NO:14, or the complement thereof; b) labeling the amplified nucleic acid molecule with a detectable label; c) contacting the labeled nucleic acid molecule with a support comprising an alteration-specific probe, wherein the alteration-specific probe comprises a nucleotide sequence which hybridizes under stringent conditions to the nucleic acid sequence of the amplified nucleic acid molecule comprising: a uracil at a position corresponding to position 553 according to SEQ ID NO:8, or the complement thereof; and/or a thymine at a position corresponding to position 553 according to SEQ ID NO:14, or the complement thereof; and d) detecting the detectable label.

In some embodiments, the determining step, detecting step, or sequence analysis comprises: a) amplifying at least a portion of the nucleic acid molecule that encodes the human FSCN2 polypeptide, wherein the amplified portion comprises: a uracil at a position corresponding to position 567 according to SEQ ID NO:20, or the complement thereof; and/or a thymine at a position corresponding to position 567 according to SEQ ID NO:22, or the complement thereof; b) labeling the amplified nucleic acid molecule with a detectable label; c) contacting the labeled nucleic acid molecule with a support comprising an alteration-specific probe, wherein the alteration-specific probe comprises a nucleotide sequence which hybridizes under stringent conditions to the nucleic acid sequence of the amplified nucleic acid molecule comprising: a uracil at a position corresponding to position 567 according to SEQ ID NO:20, or the complement thereof; and/or a thymine at a position corresponding to position 567 according to SEQ ID NO:22, or the complement thereof; and d) detecting the detectable label.

In some embodiments, the determining step, detecting step, or sequence analysis comprises: a) amplifying at least a portion of the nucleic acid molecule that encodes the human FSCN2 polypeptide, wherein the amplified portion comprises a thymine at a position corresponding to position 548 according to SEQ ID NO:2, or the complement thereof; b) labeling the amplified nucleic acid molecule with a detectable label; c) contacting the labeled nucleic acid molecule with a support comprising an alteration-specific probe, wherein the alteration-specific probe comprises a nucleotide sequence which hybridizes under stringent conditions to the nucleic acid sequence of the amplified nucleic acid molecule comprising a thymine at a position corresponding to position 548 according to SEQ ID NO:2, or the complement thereof; and d) detecting the detectable label.

In some embodiments, the determining step, detecting step, or sequence analysis comprises: a) amplifying at least a portion of the nucleic acid molecule that encodes the human FSCN2 polypeptide, wherein the amplified portion comprises: a uracil at a position corresponding to position 548 according to SEQ ID NO:6, or the complement thereof; a uracil at a position corresponding to position 469 according to SEQ ID NO:7, or the complement thereof; a uracil at a position corresponding to position 553 according to SEQ ID NO:8, or the complement thereof; or a uracil at a position corresponding to position 567 according to SEQ ID NO:20, or the complement thereof; b) labeling the amplified nucleic acid molecule with a detectable label; c) contacting the labeled nucleic acid molecule with a support comprising an alteration-specific probe, wherein the alteration-specific probe comprises a nucleotide sequence which hybridizes under stringent conditions to the nucleic acid sequence of the amplified nucleic acid molecule comprising: a uracil at a position corresponding to position 548 according to SEQ ID NO:6, or the complement thereof; a uracil at a position corresponding to position 469 according to SEQ ID NO:7, or the complement thereof; a uracil at a position corresponding to position 553 according to SEQ ID NO:8, or the complement thereof; or a uracil at a position corresponding to position 567 according to SEQ ID NO:20, or the complement thereof.

In some embodiments, the determining step, detecting step, or sequence analysis comprises: a) amplifying at least a portion of the nucleic acid molecule that encodes the human FSCN2 polypeptide, wherein the amplified portion comprises: a thymine at a position corresponding to position 548 according to SEQ ID NO:12, or the complement thereof; a thymine at a position corresponding to position 469 according to SEQ ID NO:13, or the complement thereof; a thymine at a position corresponding to position 553 according to SEQ ID NO:14, or the complement thereof; or a thymine at a position corresponding to position 567 according to SEQ ID NO:22, or the complement thereof;b) labeling the amplified nucleic acid molecule with a detectable label; c) contacting the labeled nucleic acid molecule with a support comprising an alteration-specific probe, wherein the alteration-specific probe comprises a nucleotide sequence which hybridizes under stringent conditions to the nucleic acid sequence of the amplified nucleic acid molecule comprising: a thymine at a position corresponding to position 548 according to SEQ ID NO:12, or the complement thereof; a thymine at a position corresponding to position 469 according to SEQ ID NO:13, or the complement thereof; a thymine at a position corresponding to position 553 according to SEQ ID NO:14, or the complement thereof; or a thymine at a position corresponding to position 567 according to SEQ ID NO:22, or the complement thereof;and d) detecting the detectable label.

In some embodiments, the nucleic acid molecule is mRNA and the determining step further comprises reverse-transcribing the mRNA into a cDNA prior to the amplifying step.

In some embodiments, the determining step, detecting step, or sequence analysis comprises: contacting the nucleic acid molecule in the biological sample with an alteration-specific probe comprising a detectable label, wherein the alteration-specific probe comprises a nucleotide sequence which hybridizes under stringent conditions to the nucleotide sequence of the amplified nucleic acid molecule comprising: a thymine at a position corresponding to position 548 according to SEQ ID NO:2, or the complement thereof; a uracil at a position corresponding to position 548 according to SEQ ID NO:6, or the complement thereof; and/or a thymine at a position corresponding to position 548 according to SEQ ID NO:12, or the complement thereof; and detecting the detectable label.

In some embodiments, the determining step, detecting step, or sequence analysis comprises: contacting the nucleic acid molecule in the biological sample with an alteration-specific probe comprising a detectable label, wherein the alteration-specific probe comprises a nucleotide sequence which hybridizes under stringent conditions to the nucleotide sequence of the amplified nucleic acid molecule comprising: a uracil at a position corresponding to position 469 according to SEQ ID NO:7, or the complement thereof; and/or a thymine at a position corresponding to position 469 according to SEQ ID NO:13, or the complement thereof; and detecting the detectable label.

In some embodiments, the determining step, detecting step, or sequence analysis comprises: contacting the nucleic acid molecule in the biological sample with an alteration-specific probe comprising a detectable label, wherein the alteration-specific probe comprises a nucleotide sequence which hybridizes under stringent conditions to the nucleotide sequence of the amplified nucleic acid molecule comprising: a uracil at a position corresponding to position 553 according to SEQ ID NO:8, or the complement thereof; and/or a thymine at a position corresponding to position 553 according to SEQ ID NO:14, or the complement thereof; and detecting the detectable label.

In some embodiments, the determining step, detecting step, or sequence analysis comprises: contacting the nucleic acid molecule in the biological sample with an alteration-specific probe comprising a detectable label, wherein the alteration-specific probe comprises a nucleotide sequence which hybridizes under stringent conditions to the nucleotide sequence of the amplified nucleic acid molecule comprising: a uracil at a position corresponding to position 567 according to SEQ ID NO:20, or the complement thereof; and/or a thymine at a position corresponding to position 567 according to SEQ ID NO:22, or the complement thereof; and detecting the detectable label.

In some embodiments, the determining step, detecting step, or sequence analysis comprises: contacting the nucleic acid molecule in the biological sample with an alteration-specific probe comprising a detectable label, wherein the alteration-specific probe comprises a nucleotide sequence which hybridizes under stringent conditions to the nucleotide sequence of the amplified nucleic acid molecule comprising a thymine at a position corresponding to position 548 according to SEQ ID NO:2, or the complement thereof and detecting the detectable label.

In some embodiments, the determining step, detecting step, or sequence analysis comprises: contacting the nucleic acid molecule in the biological sample with an alteration-specific probe comprising a detectable label, wherein the alteration-specific probe comprises a nucleotide sequence which hybridizes under stringent conditions to the nucleotide sequence of the amplified nucleic acid molecule comprising: a uracil at a position corresponding to position 548 according to SEQ ID NO:6, or the complement thereof; a uracil at a position corresponding to position 469 according to SEQ ID NO:7, or the complement thereof; a uracil at a position corresponding to position 553 according to SEQ ID NO:8, or the complement thereof; or a uracil at a position corresponding to position 567 according to SEQ ID NO:20, or the complement thereof; and detecting the detectable label.

In some embodiments, the determining step, detecting step, or sequence analysis comprises: contacting the nucleic acid molecule in the biological sample with an alteration-specific probe comprising a detectable label, wherein the alteration-specific probe comprises a nucleotide sequence which hybridizes under stringent conditions to the nucleotide sequence of the amplified nucleic acid molecule comprising: a thymine at a position corresponding to position 548 according to SEQ ID NO:12, or the complement thereof; a thymine at a position corresponding to position 469 according to SEQ ID NO:13, or the complement thereof; a thymine at a position corresponding to position 553 according to SEQ ID NO:14, or the complement thereof; or a thymine at a position corresponding to position 567 according to SEQ ID NO:22, or the complement thereof; and detecting the detectable label.

Alteration-specific polymerase chain reaction techniques can be used to detect mutations such as SNPs in a nucleic acid sequence. Alteration-specific primers can be used because the DNA polymerase will not extend when a mismatch with the template is present.

In some embodiments, the nucleic acid molecule in the sample is mRNA and the mRNA is reverse-transcribed into a cDNA prior to the amplifying step. In some embodiments, the nucleic acid molecule is present within a cell obtained from the subject.

In some embodiments, the assay comprises contacting the biological sample with a primer or probe, such as an alteration-specific primer or alteration-specific probe, that specifically hybridizes to an FSCN2 variant genomic sequence, variant mRNA sequence, or variant cDNA sequence and not the corresponding FSCN2 reference sequence under stringent conditions, and determining whether hybridization has occurred.

In some embodiments, the assay comprises RNA sequencing (RNA-Seq). In some embodiments, the assays also comprise reverse transcribing mRNA into cDNA, such as by the reverse transcriptase polymerase chain reaction (RT-PCR).

In some embodiments, the methods utilize probes and primers of sufficient nucleotide length to bind to the target nucleotide sequence and specifically detect and/or identify a polynucleotide comprising an FSCN2 variant genomic nucleic acid molecule, variant mRNA molecule, or variant cDNA molecule. The hybridization conditions or reaction conditions can be determined by the operator to achieve this result. The nucleotide length may be any length that is sufficient for use in a detection method of choice, including any assay described or exemplified herein. Such probes and primers can hybridize specifically to a target nucleotide sequence under high stringency hybridization conditions. Probes and primers may have complete nucleotide sequence identity of contiguous nucleotides within the target nucleotide sequence, although probes differing from the target nucleotide sequence and that retain the ability to specifically detect and/or identify a target nucleotide sequence may be designed by conventional methods. Probes and primers can have about 80%, about 85%, about 90%, about 91%, about 92%, about 93%, about 94%, about 95%, about 96%, about 97%, about 98%, about 99%, or 100% sequence identity or complementarity with the nucleotide sequence of the target nucleic acid molecule.

In some embodiments, to determine whether an FSCN2 nucleic acid molecule (genomic nucleic acid molecule, mRNA molecule, or cDNA molecule), or complement thereof, within a biological sample comprises a nucleotide sequence comprising a thymine at a position corresponding to position 548 according to SEQ ID NO:2 (genomic nucleic acid molecule), or a uracil at a position corresponding to position 548 according to SEQ ID NO:6 (mRNA molecule), or a thymine at a position corresponding to position 548 according to SEQ ID NO:12 (cDNA molecule), the biological sample can be subjected to an amplification method using a primer pair that includes a first primer derived from the 5' flanking sequence adjacent to a thymine at a position corresponding to position 548 according to SEQ ID NO:2, or a uracil at a position corresponding to position 548 according to SEQ ID NO:6, or a thymine at a position corresponding to position 548 according to SEQ ID NO:12, and a second primer derived from the 3' flanking sequence adjacent to a thymine at a position corresponding to position 548 according to SEQ ID NO:2, or a uracil at a position corresponding to position 548 according to SEQ ID NO:6, or a thymine at a position corresponding to position 548 according to SEQ ID NO:12 to produce an amplicon that is indicative of the presence of the SNP at positions encoding a thymine at a position corresponding to position 548 according to SEQ ID NO:2, or a uracil at a position corresponding to position 548 according to SEQ ID NO:6, or a thymine at a position corresponding to position 548 according to SEQ ID NO:12. In some embodiments, the amplicon may range in length from the combined length of the primer pairs plus one nucleotide base pair to any length of amplicon producible by a DNA amplification protocol. This distance can range from one nucleotide base pair up to the limits of the amplification reaction, or about twenty thousand nucleotide base pairs. Optionally, the primer pair flanks a region including positions comprising a thymine at a position corresponding to position 548 according to SEQ ID NO:2, or a uracil at a position corresponding to position 548 according to SEQ ID NO:6, or a thymine at a position corresponding to position 548 according to SEQ ID NO:12, and at least 1, 2, 3, 4, 5, 6, 7, 8, 9, 10, or more nucleotides on each side of positions comprising a thymine at a position corresponding to position 548 according to SEQ ID NO:2, or a uracil at a position corresponding to position 548 according to SEQ ID NO:6, or a thymine at a position corresponding to position 548 according to SEQ ID NO:12.

In some embodiments, to determine whether an FSCN2 nucleic acid molecule (genomic nucleic acid molecule, mRNA molecule, or cDNA molecule), or complement thereof, within a biological sample comprises a nucleotide sequence comprising a uracil at a position corresponding to position 469 according to SEQ ID NO:7 (mRNA molecule), or a thymine at a position corresponding to position 469 according to SEQ ID NO:13 (cDNA molecule), the biological sample can be subjected to an amplification method using a primer pair that includes a first primer derived from the 5' flanking sequence adjacent to a uracil at a position corresponding to position 469 according to SEQ ID NO:7, or a thymine at a position corresponding to position 469 according to SEQ ID NO:13, and a second primer derived from the 3' flanking sequence adjacent to a uracil at a position corresponding to position 469 according to SEQ ID NO:7, or a thymine at a position corresponding to position 469 according to SEQ ID NO:13 to produce an amplicon that is indicative of the presence of the SNP at positions encoding a uracil at a position corresponding to position 469 according to SEQ ID NO:7, or a thymine at a position corresponding to position 469 according to SEQ ID NO:13. In some embodiments, the amplicon may range in length from the combined length of the primer pairs plus one nucleotide base pair to any length of amplicon producible by a DNA amplification protocol. This distance can range from one nucleotide base pair up to the limits of the amplification reaction, or about twenty thousand nucleotide base pairs. Optionally, the primer pair flanks a region including positions comprising a uracil at a position corresponding to position 469 according to SEQ ID NO:7, or a thymine at a position corresponding to position 469 according to SEQ ID NO:13, and at least 1, 2, 3, 4, 5, 6, 7, 8, 9, 10, or more nucleotides on each side of positions comprising a uracil at a position corresponding to position 469 according to SEQ ID NO:7, or a thymine at a position corresponding to position 469 according to SEQ ID NO:13.

In some embodiments, to determine whether an FSCN2 nucleic acid molecule (genomic nucleic acid molecule, mRNA molecule, or cDNA molecule), or complement thereof, within a biological sample comprises a nucleotide sequence comprising a uracil at a position corresponding to position 553 according to SEQ ID NO:8 (mRNA molecule), or a thymine at a position corresponding to position 553 according to SEQ ID NO:14 (cDNA molecule), the biological sample can be subjected to an amplification method using a primer pair that includes a first primer derived from the 5' flanking sequence adjacent to a uracil at a position corresponding to position 553 according to SEQ ID NO:8, or a thymine at a position corresponding to position 553 according to SEQ ID NO:14, and a second primer derived from the 3' flanking sequence adjacent to a uracil at a position corresponding to position 553 according to SEQ ID NO:8, or a thymine at a position corresponding to position 553 according to SEQ ID NO:14 to produce an amplicon that is indicative of the presence of the SNP at positions encoding a uracil at a position corresponding to position 553 according to SEQ ID NO:8, or a thymine at a position corresponding to position 553 according to SEQ ID NO:14. In some embodiments, the amplicon may range in length from the combined length of the primer pairs plus one nucleotide base pair to any length of amplicon producible by a DNA amplification protocol. This distance can range from one nucleotide base pair up to the limits of the amplification reaction, or about twenty thousand nucleotide base pairs. Optionally, the primer pair flanks a region including positions comprising a uracil at a position corresponding to position 553 according to SEQ ID NO:8, or a thymine at a position corresponding to position 553 according to SEQ ID NO:14, and at least 1, 2, 3, 4, 5, 6, 7, 8, 9, 10, or more nucleotides on each side of positions comprising a uracil at a position corresponding to position 553 according to SEQ ID NO:8, or a thymine at a position corresponding to position 553 according to SEQ ID NO:14.

In some embodiments, to determine whether an FSCN2 nucleic acid molecule (genomic nucleic acid molecule, mRNA molecule, or cDNA molecule), or complement thereof, within a biological sample comprises a nucleotide sequence comprising a uracil at a position corresponding to position 567 according to SEQ ID NO:20 (mRNA molecule), or a thymine at a position corresponding to position 567 according to SEQ ID NO:22 (cDNA molecule), the biological sample can be subjected to an amplification method using a primer pair that includes a first primer derived from the 5' flanking sequence adjacent to a uracil at a position corresponding to position 567 according to SEQ ID NO:20, or a thymine at a position corresponding to position 567 according to SEQ ID NO:22, and a second primer derived from the 3' flanking sequence adjacent to a uracil at a position corresponding to position 567 according to SEQ ID NO:20, or a thymine at a position corresponding to position 567 according to SEQ ID NO:22 to produce an amplicon that is indicative of the presence of the SNP at positions encoding a uracil at a position corresponding to position 567 according to SEQ ID NO:20, or a thymine at a position corresponding to position 567 according to SEQ ID NO:22. In some embodiments, the amplicon may range in length from the combined length of the primer pairs plus one nucleotide base pair to any length of amplicon producible by a DNA amplification protocol. This distance can range from one nucleotide base pair up to the limits of the amplification reaction, or about twenty thousand nucleotide base pairs. Optionally, the primer pair flanks a region including positions comprising a uracil at a position corresponding to position 567 according to SEQ ID NO:20, or a thymine at a position corresponding to position 567 according to SEQ ID NO:22, and at least 1, 2, 3, 4, 5, 6, 7, 8, 9, 10, or more nucleotides on each side of positions comprising a uracil at a position corresponding to position 567 according to SEQ ID NO:20, or a thymine at a position corresponding to position 567 according to SEQ ID NO:22.

Similar amplicons can be generated from the mRNA and/or cDNA sequences. PCR primer pairs can be derived from a known sequence, for example, by using computer programs intended for that purpose, such as the PCR primer analysis tool in Vector NTI version 10 (Informax Inc., Bethesda Md.); PrimerSelect (DNASTAR Inc., Madison, Wis.); and Primer3 (Version 0.4.0.COPYRGT., 1991, Whitehead Institute for Biomedical Research, Cambridge, Mass.). Additionally, the sequence can be visually scanned and primers manually identified using known guidelines.

Illustrative examples of nucleic acid sequencing techniques include, but are not limited to, chain terminator (Sanger) sequencing and dye terminator sequencing. Other methods involve nucleic acid hybridization methods other than sequencing, including using labeled primers or probes directed against purified DNA, amplified DNA, and fixed cell preparations (fluorescence in situ hybridization (FISH)). In some methods, a target nucleic acid molecule may be amplified prior to or simultaneous with detection. Illustrative examples of nucleic acid amplification techniques include, but are not limited to, polymerase chain reaction (PCR), ligase chain reaction (LCR), strand displacement amplification (SDA), and nucleic acid sequence based amplification (NASBA). Other methods include, but are not limited to, ligase chain reaction, strand displacement amplification, and thermophilic SDA (tSDA).

In hybridization techniques, stringent conditions can be employed such that a probe or primer will specifically hybridize to its target. In some embodiments, a polynucleotide primer or probe under stringent conditions will hybridize to its target sequence to a detectably greater degree than to other non-target sequences, such as, at least 2-fold, at least 3-fold, at least 4-fold, or more over background, including over 10-fold over background. In some embodiments, a polynucleotide primer or probe under stringent conditions will hybridize to its target nucleotide sequence to a detectably greater degree than to other nucleotide sequences by at least 2-fold. In some embodiments, a polynucleotide primer or probe under stringent conditions will hybridize to its target nucleotide sequence to a detectably greater degree than to other nucleotide sequences by at least 3-fold. In some embodiments, a polynucleotide primer or probe under stringent conditions will hybridize to its target nucleotide sequence to a detectably greater degree than to other nucleotide sequences by at least 4-fold. In some embodiments, a polynucleotide primer or probe under stringent conditions will hybridize to its target nucleotide sequence to a detectably greater degree than to other nucleotide sequences by over 10-fold over background. Stringent conditions are sequence-dependent and will be different in different circumstances.

Appropriate stringency conditions which promote DNA hybridization, for example, 6X sodium chloride/sodium citrate (SSC) at about 45°C., followed by a wash of 2X SSC at 50°C, are known or can be found in Current Protocols in Molecular Biology, John Wiley & Sons, N.Y. (1989), 6.3.1-6.3.6. Typically, stringent conditions for hybridization and detection will be those in which the salt concentration is less than about 1.5 M Na⁺ ion, typically about 0.01 to 1.0 M Na⁺ ion concentration (or other salts) at pH 7.0 to 8.3 and the temperature is at least about 30°C for short probes (such as, for example, 10 to 50 nucleotides) and at least about 60°C for longer probes (such as, for example, greater than 50 nucleotides). Stringent conditions may also be achieved with the addition of destabilizing agents such as formamide. Optionally, wash buffers may comprise about 0.1% to about 1% SDS. Duration of hybridization is generally less than about 24 hours, usually about 4 to about 12 hours. The duration of the wash time will be at least a length of time sufficient to reach equilibrium.

The present disclosure also provides methods of detecting the presence of a human FSCN2 predicted loss-of-function polypeptide comprising performing an assay on a sample obtained from a subject to determine whether an FSCN2 polypeptide in the subject contains one or more variations that causes the polypeptide to have a loss-of-function (partial or complete) or predicted loss-of-function (partial or complete). The FSCN2 predicted loss-of-function polypeptide can be any of the FSCN2 truncated variant polypeptides described herein. In some embodiments, the methods detect the presence of an FSCN2 H138Y polypeptide. In some embodiments, the methods detect the presence of FSCN2 H138Y Long or FSCN2 H138Y Short.

In some embodiments, the methods comprise performing an assay on a sample obtained from a subject to determine whether an FSCN2 polypeptide in the sample comprises a tyrosine at a position corresponding to position 138 according to SEQ ID NO:17. In some embodiments, the methods comprise performing an assay on a sample obtained from a subject to determine whether an FSCN2 polypeptide in the sample comprises a tyrosine at a position corresponding to position 138 according to SEQ ID NO:18.

In some embodiments, the detecting step comprises sequencing at least a portion of the polypeptide that comprises a position corresponding to position 138 according to SEQ ID NO:17, SEQ ID NO:15, or SEQ ID NO:16. In some embodiments, the detecting step comprises sequencing at least a portion of the polypeptide that comprises a position corresponding to position 138 according to SEQ ID NO:18 or SEQ ID NO:15 or SEQ ID NO:16.

In some embodiments, the detecting step comprises an immunoassay for detecting the presence of a polypeptide that comprises a position corresponding to position 138 according to SEQ ID NO:17, SEQ ID NO:15, or SEQ ID NO:16. In some embodiments, the detecting step comprises an immunoassay for detecting the presence of a polypeptide that comprises a position corresponding to position 138 according to SEQ ID NO:18 or SEQ ID NO:15 or SEQ ID NO:16.

In some embodiments, when the subject does not have an FSCN2 predicted loss-of-function polypeptide, then the subject does not have an increased risk for developing hearing loss or any of conductive hearing loss, sensorineural hearing loss, or neural hearing loss. In some embodiments, when the subject has an FSCN2 predicted loss-of-function polypeptide, then the subject has an increased risk for developing hearing loss or any of conductive hearing loss, sensorineural hearing loss, or neural hearing loss.

The present disclosure also provides isolated nucleic acid molecules that hybridize to FSCN2 variant genomic nucleic acid molecules, FSCN2 variant mRNA molecules, and/or FSCN2 variant cDNA molecules (such as any of the genomic variant nucleic acid molecules, mRNA variant molecules, and cDNA variant molecules disclosed herein). In some embodiments, the isolated nucleic acid molecules hybridize to a portion of the FSCN2 nucleic acid molecule that includes a position corresponding to: position 548 according to SEQ ID NO:2, position 548 according to SEQ ID NO:6, or position 548 according to SEQ ID NO:12. In some embodiments, the isolated nucleic acid molecules hybridize to a portion of the FSCN2 nucleic acid molecule that includes a position corresponding to position 469 according to SEQ ID NO:7, or position 469 according to SEQ ID NO:13. In some embodiments, the isolated nucleic acid molecules hybridize to a portion of the FSCN2 nucleic acid molecule that includes a position correspondingposition 553 according to SEQ ID NO:8, or position 553 according to SEQ ID NO:14. In some embodiments, the isolated nucleic acid molecules hybridize to a portion of the FSCN2 nucleic acid molecule that includes a position corresponding to position 567 according to SEQ ID NO:20, or position 567 according to SEQ ID NO:22.

In some embodiments, such isolated nucleic acid molecules comprise or consist of at least about 5, at least about 8, at least about 10, at least about 11, at least about 12, at least about 13, at least about 14, at least about 15, at least about 16, at least about 17, at least about 18, at least about 19, at least about 20, at least about 21, at least about 22, at least about 23, at least about 24, at least about 25, at least about 30, at least about 35, at least about 40, at least about 45, at least about 50, at least about 55, at least about 60, at least about 65, at least about 70, at least about 75, at least about 80, at least about 85, at least about 90, at least about 95, at least about 100, at least about 200, at least about 300, at least about 400, at least about 500, at least about 600, at least about 700, at least about 800, at least about 900, at least about 1000, at least about 2000, at least about 3000, at least about 4000, or at least about 5000 nucleotides. In some embodiments, such isolated nucleic acid molecules comprise or consist of at least about 5, at least about 8, at least about 10, at least about 11, at least about 12, at least about 13, at least about 14, at least about 15, at least about 16, at least about 17, at least about 18, at least about 19, at least about 20, at least about 21, at least about 22, at least about 23, at least about 24, or at least about 25 nucleotides. In some embodiments, the isolated nucleic acid molecules comprise or consist of at least about 18 nucleotides. In some embodiments, the isolated nucleic acid molecules comprise or consists of at least about 15 nucleotides. In some embodiments, the isolated nucleic acid molecules consist of or comprise from about 10 to about 35, from about 10 to about 30, from about 10 to about 25, from about 12 to about 30, from about 12 to about 28, from about 12 to about 24, from about 15 to about 30, from about 15 to about 25, from about 18 to about 30, from about 18 to about 25, from about 18 to about 24, or from about 18 to about 22 nucleotides. In some embodiments, the isolated nucleic acid molecules consist of or comprise from about 18 to about 30 nucleotides. In some embodiments, the isolated nucleic acid molecules comprise or consist of at least about 15 nucleotides to at least about 35 nucleotides.

In some embodiments, such isolated nucleic acid molecules hybridize to FSCN2 variant nucleic acid molecules (such as genomic nucleic acid molecules, mRNA molecules, and/or cDNA molecules) under stringent conditions. Such nucleic acid molecules can be used, for example, as probes, primers, alteration-specific probes, or alteration-specific primers as described or exemplified herein, and include, without limitation primers, probes, antisense RNAs, shRNAs, and siRNAs, each of which is described in more detail elsewhere herein, and can be used in any of the methods described herein.

In some embodiments, the isolated nucleic acid molecules hybridize to at least about 15 contiguous nucleotides of a nucleic acid molecule that is at least about 70%, at least about 75%, at least about 80%, at least about 85%, at least about 90%, at least about 95%, at least about 96%, at least about 97%, at least about 98%, at least about 99%, or 100% identical to FSCN2 variant genomic nucleic acid molecules, FSCN2 variant mRNA molecules, and/or FSCN2 variant cDNA molecules. In some embodiments, the isolated nucleic acid molecules consist of or comprise from about 15 to about 100 nucleotides, or from about 15 to about 35 nucleotides. In some embodiments, the isolated nucleic acid molecules consist of or comprise from about 15 to about 100 nucleotides. In some embodiments, the isolated nucleic acid molecules consist of or comprise from about 15 to about 35 nucleotides.

In some embodiments, the isolated alteration-specific probes or alteration-specific primers comprise at least about 15 nucleotides, wherein the alteration-specific probe or alteration-specific primer comprises a nucleotide sequence which is complementary to a portion of a nucleotide sequence encoding a human FSCN2 polypeptide, wherein the portion comprises a position corresponding to: position 548 according to SEQ ID NO:2, or the complement thereof; position 548 according to SEQ ID NO:6, or the complement thereof; or position 548 according to SEQ ID NO:12, or the complement thereof. In some embodiments, the alteration-specific probe or alteration-specific primer comprises a nucleotide sequence which is complementary to a portion of a nucleotide sequence comprising positions corresponding to: positions 548-550 according to SEQ ID NO:2, or the complement thereof; positions 548-550 according to SEQ ID NO:6, or the complement thereof; and/or positions 548-550 according to SEQ ID NO:12, or the complement thereof.

In some embodiments, the isolated alteration-specific probes or alteration-specific primers comprise at least about 15 nucleotides, wherein the alteration-specific probe or alteration-specific primer comprises a nucleotide sequence which is complementary to a portion of a nucleotide sequence encoding a human FSCN2 polypeptide, wherein the portion comprises a position corresponding to position 469 according to SEQ ID NO:7, or the complement thereof; or position 469 according to SEQ ID NO:13, or the complement thereof. In some embodiments, the alteration-specific probe or alteration-specific primer comprises a nucleotide sequence which is complementary to a portion of a nucleotide sequence comprising positions corresponding to positions 469-471 according to SEQ ID NO:7, or the complement thereof; and/or positions 469-471 according to SEQ ID NO:13, or the complement thereof.

In some embodiments, the isolated alteration-specific probes or alteration-specific primers comprise at least about 15 nucleotides, wherein the alteration-specific probe or alteration-specific primer comprises a nucleotide sequence which is complementary to a portion of a nucleotide sequence encoding a human FSCN2 polypeptide, wherein the portion comprises a position corresponding to position 553 according to SEQ ID NO:8, or the complement thereof; or position 553 according to SEQ ID NO:14, or the complement thereof. In some embodiments, the alteration-specific probe or alteration-specific primer comprises a nucleotide sequence which is complementary to a portion of a nucleotide sequence comprising positions corresponding to positions 553-555 according to SEQ ID NO:8, or the complement thereof; and/or positions 553-555 according to SEQ ID NO:14, or the complement thereof.

In some embodiments, the isolated alteration-specific probes or alteration-specific primers comprise at least about 15 nucleotides, wherein the alteration-specific probe or alteration-specific primer comprises a nucleotide sequence which is complementary to a portion of a nucleotide sequence encoding a human FSCN2 polypeptide, wherein the portion comprises a position corresponding to position 567 according to SEQ ID NO:20, or the complement thereof; or position 567 according to SEQ ID NO:22, or the complement thereof. In some embodiments, the alteration-specific probe or alteration-specific primer comprises a nucleotide sequence which is complementary to a portion of a nucleotide sequence comprising positions corresponding to positions 567-569 according to SEQ ID NO:20, or the complement thereof; and/or positions 567-569 according to SEQ ID NO:22, or the complement thereof.

In some embodiments, the alteration-specific probes and alteration-specific primers comprise DNA. In some embodiments, the alteration-specific probes and alteration-specific primers comprise RNA.

In some embodiments, the probes and primers described herein (including alteration-specific probes and alteration-specific primers) have a nucleotide sequence that specifically hybridizes to any of the nucleic acid molecules disclosed herein, or the complement thereof. In some embodiments, the probes and primers specifically hybridize to any of the nucleic acid molecules disclosed herein under stringent conditions.

In some embodiments, the primers, including alteration-specific primers, can be used in second generation sequencing or high throughput sequencing. In some instances, the primers, including alteration-specific primers, can be modified. In particular, the primers can comprise various modifications that are used at different steps of, for example, Massive Parallel Signature Sequencing (MPSS), Polony sequencing, and 454 Pyrosequencing. Modified primers can be used at several steps of the process, including biotinylated primers in the cloning step and fluorescently labeled primers used at the bead loading step and detection step. Polony sequencing is generally performed using a paired-end tags library wherein each molecule of DNA template is about 135 bp in length. Biotinylated primers are used at the bead loading step and emulsion PCR. Fluorescently labeled degenerate nonamer oligonucleotides are used at the detection step. An adaptor can contain a 5'-biotin tag for immobilization of the DNA library onto streptavidin-coated beads.

The probes and primers described herein can be used to detect a nucleotide variation within any of the FSCN2 variant genomic nucleic acid molecules, FSCN2 variant mRNA molecules, and/or FSCN2 variant cDNA molecules disclosed herein. The primers described herein can be used to amplify FSCN2 variant genomic nucleic acid molecules, FSCN2 variant mRNA molecules, or FSCN2 variant cDNA molecules, or a fragment thereof.

The present disclosure also provides pairs of primers comprising any of the primers described above. For example, if one of the primers' 3'-ends hybridizes to a cytosine at a position coresponding to position 548 according to SEQ ID NO:1 (rather than thymine) in a particular FSCN2 nucleic acid molecule, then the presence of the amplified fragment would indicate the presence of an FSCN2 reference genomic nucleic acid molecule. Conversely, if one of the primers' 3'-ends hybridizes to a thymine at a position corresponding to position 548 according to SEQ ID NO:2 (rather than cytosine) in a particular FSCN2 nucleic acid molecule, then the presence of the amplified fragment would indicate the presence of the FSCN2 variant genomic nucleic acid molecule. In some embodiments, the nucleotide of the primer complementary to the thymine at a position corresponding to position 548 according to SEQ ID NO:2 can be at the 3' end of the primer. In addition, if one of the primers' 3'-ends hybridizes to a cytosine at a position corresponding to position 548 according to SEQ ID NO:3 (rather than uracil) in a particular FSCN2 nucleic acid molecule, then the presence of the amplified fragment would indicate the presence of an FSCN2 reference mRNA molecule. Conversely, if one of the primers' 3'-ends hybridizes to a uracil at a position corresponding to position 548 according to SEQ ID NO:6 (rather than cytosine) in a particular FSCN2 mRNA molecule, then the presence of the amplified fragment would indicate the presence of the FSCN2 variant mRNA molecule. In some embodiments, the nucleotide of the primer complementary to the uracil at a position corresponding to position 548 according to SEQ ID NO:6 can be at the 3' end of the primer. In addition, if one of the primers' 3'-ends hybridizes to a cytosine at a position corresponding to position 548 according to SEQ ID NO:9 (rather than thymine) in a particular FSCN2 nucleic acid molecule, then the presence of the amplified fragment would indicate the presence of an FSCN2 reference cDNA molecule. Conversely, if one of the primers' 3'-ends hybridizes to a thymine at a position corresponding to position 548 according to SEQ ID NO:12 (rather than cytosine) in a particular FSCN2 cDNA molecule, then the presence of the amplified fragment would indicate the presence of the FSCN2 variant cDNA molecule. In some embodiments, the nucleotide of the primer complementary to the thymine at a position corresponding to position 548 according to SEQ ID NO:12 can be at the 3' end of the primer.

If, for example, one of the primers' 3'-ends hybridizes to a cytosine at a position corresponding to position 469 according to SEQ ID NO:4 (rather than uracil) in a particular FSCN2 nucleic acid molecule, then the presence of the amplified fragment would indicate the presence of an FSCN2 reference mRNA molecule. Conversely, if one of the primers' 3'-ends hybridizes to a uracil at a position corresponding to position 469 according to SEQ ID NO:7 (rather than cytosine) in a particular FSCN2 mRNA molecule, then the presence of the amplified fragment would indicate the presence of the FSCN2 variant mRNA molecule. In some embodiments, the nucleotide of the primer complementary to the uracil at a position corresponding to position 469 according to SEQ ID NO:7 can be at the 3' end of the primer. In addition, if one of the primers' 3'-ends hybridizes to a cytosine at a position corresponding to position 469 according to SEQ ID NO:10 (rather than thymine) in a particular FSCN2 nucleic acid molecule, then the presence of the amplified fragment would indicate the presence of an FSCN2 reference cDNA molecule. Conversely, if one of the primers' 3'-ends hybridizes to a thymine at a position corresponding to position 469 according to SEQ ID NO:13 (rather than cytosine) in a particular FSCN2 cDNA molecule, then the presence of the amplified fragment would indicate the presence of the FSCN2 variant cDNA molecule. In some embodiments, the nucleotide of the primer complementary to the thymine at a position corresponding to position 469 according to SEQ ID NO:13 can be at the 3' end of the primer.

If, for example, one of the primers' 3'-ends hybridizes to a cytosine at a position corresponding to position 553 according to SEQ ID NO:5 (rather than uracil) in a particular FSCN2 nucleic acid molecule, then the presence of the amplified fragment would indicate the presence of an FSCN2 reference mRNA molecule. Conversely, if one of the primers' 3'-ends hybridizes to a uracil at a position corresponding to position 553 according to SEQ ID NO:8 (rather than cytosine) in a particular FSCN2 mRNA molecule, then the presence of the amplified fragment would indicate the presence of the FSCN2 variant mRNA molecule. In some embodiments, the nucleotide of the primer complementary to the uracil at a position corresponding to position 553 according to SEQ ID NO:8 can be at the 3' end of the primer. In addition, if one of the primers' 3'-ends hybridizes to a cytosine at a position corresponding to position 553 according to SEQ ID NO:11 (rather than thymine) in a particular FSCN2 nucleic acid molecule, then the presence of the amplified fragment would indicate the presence of an FSCN2 reference cDNA molecule. Conversely, if one of the primers' 3'-ends hybridizes to a thymine at a position corresponding to position 553 according to SEQ ID NO:14 (rather than cytosine) in a particular FSCN2 cDNA molecule, then the presence of the amplified fragment would indicate the presence of the FSCN2 variant cDNA molecule. In some embodiments, the nucleotide of the primer complementary to the thymine at a position corresponding to position 553 according to SEQ ID NO:14 can be at the 3' end of the primer.

If, for example, one of the primers' 3'-ends hybridizes to a cytosine at a position corresponding to position 567 according to SEQ ID NO:19 (rather than uracil) in a particular FSCN2 nucleic acid molecule, then the presence of the amplified fragment would indicate the presence of an FSCN2 reference mRNA molecule. Conversely, if one of the primers' 3'-ends hybridizes to a uracil at a position corresponding to position 567 according to SEQ ID NO:20 (rather than cytosine) in a particular FSCN2 mRNA molecule, then the presence of the amplified fragment would indicate the presence of the FSCN2 variant mRNA molecule. In some embodiments, the nucleotide of the primer complementary to the uracil at a position corresponding to position 567 according to SEQ ID NO:20 can be at the 3' end of the primer. In addition, if one of the primers' 3'-ends hybridizes to a cytosine at a position corresponding to position 567 according to SEQ ID NO:21 (rather than thymine) in a particular FSCN2 nucleic acid molecule, then the presence of the amplified fragment would indicate the presence of an FSCN2 reference cDNA molecule. Conversely, if one of the primers' 3'-ends hybridizes to a thymine at a position corresponding to position 567 according to SEQ ID NO:22 (rather than cytosine) in a particular FSCN2 cDNA molecule, then the presence of the amplified fragment would indicate the presence of the FSCN2 variant cDNA molecule. In some embodiments, the nucleotide of the primer complementary to the thymine at a position corresponding to position 567 according to SEQ ID NO:22 can be at the 3' end of the primer.

In the context of the disclosure "specifically hybridizes" means that the probe or primer (such as, for example, the alteration-specific probe or alteration-specific primer) does not hybridize to a nucleic acid sequence encoding an FSCN2 reference genomic nucleic acid molecule, an FSCN2 reference mRNA molecule, and/or an FSCN2 reference cDNA molecule.

In some embodiments, the probes (such as, for example, an alteration-specific probe) comprise a label. In some embodiments, the label is a fluorescent label, a radiolabel, or biotin.

The present disclosure also provides supports comprising a substrate to which any one or more of the probes disclosed herein is attached. Solid supports are solid-state substrates or supports with which molecules, such as any of the probes disclosed herein, can be associated. A form of solid support is an array. Another form of solid support is an array detector. An array detector is a solid support to which multiple different probes have been coupled in an array, grid, or other organized pattern. A form for a solid-state substrate is a microtiter dish, such as a standard 96-well type. In some embodiments, a multiwell glass slide can be employed that normally contains one array per well.

The present disclosure also provides molecular complexes comprising or consisting of any of the FSCN2 nucleic acid molecules (genomic nucleic acid molecules, mRNA molecules, or cDNA molecules), or complement thereof, described herein and any of the alteration-specific primers or alteration-specific probes described herein. In some embodiments, the FSCN2 nucleic acid molecules (genomic nucleic acid molecules, mRNA molecules, or cDNA molecules), or complement thereof, in the molecular complexes are single-stranded. In some embodiments, the FSCN2 nucleic acid molecule is any of the genomic nucleic acid molecules described herein. In some embodiments, the FSCN2 nucleic acid molecule is any of the mRNA molecules described herein. In some embodiments, the FSCN2 nucleic acid molecule is any of the cDNA molecules described herein. In some embodiments, the molecular complex comprises or consists of any of the FSCN2 nucleic acid molecules (genomic nucleic acid molecules, mRNA molecules, or cDNA molecules), or complement thereof, described herein and any of the alteration-specific primers described herein. In some embodiments, the molecular complex comprises or consists of any of the FSCN2 nucleic acid molecules (genomic nucleic acid molecules, mRNA molecules, or cDNA molecules), or complement thereof, described herein and any of the alteration-specific probes described herein.

In some embodiments, the molecular complex comprises or consists of an alteration-specific primer or an alteration-specific probe hybridized to a genomic nucleic acid molecule comprising a nucleotide sequence encoding a human Fascin-2 polypeptide, wherein the alteration-specific primer or the alteration-specific probe is hybridized to a thymine at a position corresponding to position 548 according to SEQ ID NO:2, or the complement thereof.

In some embodiments, the molecular complex comprises or consists of an alteration-specific primer or an alteration-specific probe that is hybridized to a TAC codon at positions corresponding to positions 548-550 according to SEQ ID NO:2.

In some embodiments, the molecular complex comprises or consists of a genomic nucleic acid molecule that comprises SEQ ID NO:2.

In some embodiments, the molecular complex comprises or consists of an alteration-specific primer or an alteration-specific probe hybridized to an mRNA molecule comprising a nucleotide sequence encoding a human FSCN2 polypeptide, wherein the alteration-specific primer or the alteration-specific probe is hybridized to: a uracil at a position corresponding to position 548 according to SEQ ID NO:6, or the complement thereof; a uracil at a position corresponding to position 469 according to SEQ ID NO:7, or the complement thereof; a uracil at a position corresponding to position 553 according to SEQ ID NO:8, or the complement thereof; or a uracil at a position corresponding to position 567 according to SEQ ID NO:20, or the complement thereof.

In some embodiments, the molecular complex comprises or consists of an alteration-specific primer or an alteration-specific probe that is hybridized to: a UAC codon at positions corresponding to positions 548-550 according to SEQ ID NO:6; a UAC codon at positions corresponding to positions 469-471 according to SEQ ID NO:7; a UAC codon at positions corresponding to positions 553-555 according to SEQ ID NO:8; or a UAC codon at positions corresponding to positions 567-569 according to SEQ ID NO:20.

In some embodiments, the molecular complex comprises or consists of an mRNA molecule that comprises SEQ ID NO:6, SEQ ID NO:7, SEQ ID NO:8, or SEQ ID NO:20.

In some embodiments, the molecular complex comprises or consists of an alteration-specific primer or an alteration-specific probe hybridized to a cDNA molecule comprising a nucleotide sequence encoding a human FSCN2 polypeptide, wherein the alteration-specific primer or the alteration-specific probe is hybridized to: a thymine at a position corresponding to position 548 according to SEQ ID NO:12, or the complement thereof; a thymine at a position corresponding to position 469 according to SEQ ID NO:13, or the complement thereof; a thymine at a position corresponding to position 553 according to SEQ ID NO:14, or the complement thereof; or a thymine at a position corresponding to position 567 according to SEQ ID NO:22, or the complement thereof.

In some embodiments, the molecular complex comprises or consists of an alteration-specific primer or an alteration-specific probe that is hybridized to: a TAC codon at positions corresponding to positions 548-550 according to SEQ ID NO:12; a TAC codon at positions corresponding to positions 469-471 according to SEQ ID NO:13; a TAC codon at positions corresponding to positions 553-555 according to SEQ ID NO:14; or a TAC codon at positions corresponding to positions 567-569 according to SEQ ID NO:22.

In some embodiments, the molecular complex comprises or consists of a cDNA molecule that comprises SEQ ID NO:12, SEQ ID NO:13, SEQ ID NO:14, or SEQ ID NO:22.

In some embodiments, the molecular complex comprises an alteration-specific probe or an alteration-specific primer comprising a label. In some embodiments, the label is a fluorescent label, a radiolabel, or biotin. In some embodiments, the molecular complex further comprises a non-human polymerase.

The nucleotide sequence of an FSCN2 reference genomic nucleic acid molecule is set forth in SEQ ID NO:1. Referring to SEQ ID NO:1, position 548 is a cytosine.

A variant genomic nucleic acid molecule of FSCN2 exists, wherein the cytosine at position 548 is replaced with thymine. The nucleotide sequence of this FSCN2 variant genomic nucleic acid molecule is set forth in SEQ ID NO:2.

The nucleotide sequence of an FSCN2 reference mRNA molecule is set forth in SEQ ID NO:3. Referring to SEQ ID NO:3, position 548 is a cytosine.

The nucleotide sequence of another FSCN2 reference mRNA molecule is set forth in SEQ ID NO:4. Referring to SEQ ID NO:4, position 469 is a cytosine.

The nucleotide sequence of another FSCN2 reference mRNA molecule is set forth in SEQ ID NO:5. Referring to SEQ ID NO:5, position 553 is a cytosine.

The nucleotide sequence of another FSCN2 reference mRNA molecule is set forth in SEQ ID NO:19. Referring to SEQ ID NO:19, position 567 is a cytosine.

A variant mRNA molecule of FSCN2 exists, wherein the cytosine at position 548 is replaced with uracil. The nucleotide sequence of this FSCN2 variant mRNA molecule is set forth in SEQ ID NO:6.

Another variant mRNA molecule of FSCN2 exists, wherein the cytosine at position 469 is replaced with uracil. The nucleotide sequence of this FSCN2 variant mRNA molecule is set forth in SEQ ID NO:7.

Another variant mRNA molecule of FSCN2 exists, wherein the cytosine at position 553 is replaced with uracil. The nucleotide sequence of this FSCN2 variant mRNA molecule is set forth in SEQ ID NO:8.

Another variant mRNA molecule of FSCN2 exists, wherein the cytosine at position 567 is replaced with uracil. The nucleotide sequence of this FSCN2 variant mRNA molecule is set forth in SEQ ID NO:20.

The nucleotide sequence of an FSCN2 reference cDNA molecule is set forth in SEQ ID NO:9. Referring to SEQ ID NO:9, position 548 is a cytosine.

The nucleotide sequence of another FSCN2 reference cDNA molecule is set forth in SEQ ID NO:10. Referring to SEQ ID NO:10, position 469 is a cytosine.

The nucleotide sequence of another FSCN2 reference cDNA molecule is set forth in SEQ ID NO:11. Referring to SEQ ID NO:11, position 553 is a cytosine.

The nucleotide sequence of another FSCN2 reference cDNA molecule is set forth in SEQ ID NO:21. Referring to SEQ ID NO:21, position 567 is a cytosine.

A variant cDNA molecule of FSCN2 exists, wherein the cytosine at position 548 is replaced with thymine. The nucleotide sequence of this FSCN2 variant cDNA molecule is set forth in SEQ ID NO:12.

Another variant cDNA molecule of FSCN2 exists, wherein the cytosine at position 469 is replaced with thymine. The nucleotide sequence of this FSCN2 variant cDNA molecule is set forth in SEQ ID NO:13.

Another variant cDNA molecule of FSCN2 exists, wherein the cytosine at position 553 is replaced with thymine. The nucleotide sequence of this FSCN2 variant cDNA molecule is set forth in SEQ ID NO:14.

Another variant cDNA molecule of FSCN2 exists, wherein the cytosine at position 567 is replaced with thymine. The nucleotide sequence of this FSCN2 variant cDNA molecule is set forth in SEQ ID NO:22.

The genomic nucleic acid molecules, mRNA molecules, and cDNA molecules can be from any organism. For example, the genomic nucleic acid molecules, mRNA molecules, and cDNA molecules can be human or an ortholog from another organism, such as a non-human mammal, a rodent, a mouse, or a rat. It is understood that gene sequences within a population can vary due to polymorphisms such as single-nucleotide polymorphisms. The examples provided herein are only exemplary sequences. Other sequences are also possible.

Also provided herein are functional polynucleotides that can interact with the disclosed nucleic acid molecules. Examples of functional polynucleotides include, but are not limited to, antisense molecules, aptamers, ribozymes, triplex forming molecules, and external guide sequences. The functional polynucleotides can act as effectors, inhibitors, modulators, and stimulators of a specific activity possessed by a target molecule, or the functional polynucleotides can possess a *de novo* activity independent of any other molecules.

The isolated nucleic acid molecules disclosed herein can comprise RNA, DNA, or both RNA and DNA. The isolated nucleic acid molecules can also be linked or fused to a heterologous nucleic acid sequence, such as in a vector, or a heterologous label. For example, the isolated nucleic acid molecules disclosed herein can be within a vector or as an exogenous donor sequence comprising the isolated nucleic acid molecule and a heterologous nucleic acid sequence. The isolated nucleic acid molecules can also be linked or fused to a heterologous label. The label can be directly detectable (such as, for example, fluorophore) or indirectly detectable (such as, for example, hapten, enzyme, or fluorophore quencher). Such labels can be detectable by spectroscopic, photochemical, biochemical, immunochemical, or chemical means. Such labels include, for example, radiolabels, pigments, dyes, chromogens, spin labels, and fluorescent labels. The label can also be, for example, a chemiluminescent substance; a metal-containing substance; or an enzyme, where there occurs an enzyme-dependent secondary generation of signal. The term "label" can also refer to a "tag" or hapten that can bind selectively to a conjugated molecule such that the conjugated molecule, when added subsequently along with a substrate, is used to generate a detectable signal. For example, biotin can be used as a tag along with an avidin or streptavidin conjugate of horseradish peroxidate (HRP) to bind to the tag, and examined using a calorimetric substrate (such as, for example, tetramethylbenzidine (TMB)) or a fluorogenic substrate to detect the presence of HRP. Exemplary labels that can be used as tags to facilitate purification include, but are not limited to, myc, HA, FLAG or 3XFLAG, 6XHis or polyhistidine, glutathione-S-transferase (GST), maltose binding protein, an epitope tag, or the Fc portion of immunoglobulin. Numerous labels include, for example, particles, fluorophores, haptens, enzymes and their calorimetric, fluorogenic and chemiluminescent substrates and other labels.

The disclosed nucleic acid molecules can comprise, for example, nucleotides or non-natural or modified nucleotides, such as nucleotide analogs or nucleotide substitutes. Such nucleotides include a nucleotide that contains a modified base, sugar, or phosphate group, or that incorporates a non-natural moiety in its structure. Examples of non-natural nucleotides include, but are not limited to, dideoxynucleotides, biotinylated, aminated, deaminated, alkylated, benzylated, and fluorophor-labeled nucleotides.

The nucleic acid molecules disclosed herein can also comprise one or more nucleotide analogs or substitutions. A nucleotide analog is a nucleotide which contains a modification to either the base, sugar, or phosphate moieties. Modifications to the base moiety include, but are not limited to, natural and synthetic modifications of A, C, G, and T/U, as well as different purine or pyrimidine bases such as, for example, pseudouridine, uracil-5-yl, hypoxanthin-9-yl (I), and 2-aminoadenin-9-yl. Modified bases include, but are not limited to, 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl uracil and cytosine, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo (such as, for example, 5-bromo), 5-trifluoromethyl and other 5-substituted uracils and cytosines, 7-methylguanine, 7-methyladenine, 8-azaguanine, 8-azaadenine, 7-deazaguanine, 7-deazaadenine, 3-deazaguanine, and 3-deazaadenine.

Nucleotide analogs can also include modifications of the sugar moiety. Modifications to the sugar moiety include, but are not limited to, natural modifications of the ribose and deoxy ribose as well as synthetic modifications. Sugar modifications include, but are not limited to, the following modifications at the 2' position: OH; F; O-, S-, or N-alkyl; O-, S-, or N-alkenyl; O-, S- or N-alkynyl; or O-alkyl-O-alkyl, wherein the alkyl, alkenyl, and alkynyl may be substituted or unsubstituted C₁₋₁₀alkyl or C₂₋₁₀alkenyl, and C₂₋₁₀alkynyl. Exemplary 2' sugar modifications also include, but are not limited to, -O[(CH₂)ₙO]ₘCH₃, -O(CH₂)ₙOCH₃, -O(CH₂)ₙNH₂, -O(CH₂)ₙCH₃, -O(CH₂)ₙ-ONH₂, and -O(CH₂)ₙON[(CH₂)ₙCH₃)]₂, where n and m are from 1 to about 10. Other modifications at the 2' position include, but are not limited to, C₁₋₁₀alkyl, substituted lower alkyl, alkaryl, aralkyl, O-alkaryl or O-aralkyl, SH, SCH₃, OCN, Cl, Br, CN, CF₃, OCF₃, SOCH₃, SO₂CH₃, ONO₂, NO₂, N₃, NH₂, heterocycloalkyl, heterocycloalkaryl, aminoalkylamino, polyalkylamino, substituted silyl, an RNA cleaving group, a reporter group, an intercalator, a group for improving the pharmacokinetic properties of an oligonucleotide, or a group for improving the pharmacodynamic properties of an oligonucleotide, and other substituents having similar properties. Similar modifications may also be made at other positions on the sugar, particularly the 3' position of the sugar on the 3' terminal nucleotide or in 2'-5' linked oligonucleotides and the 5' position of 5' terminal nucleotide. Modified sugars can also include those that contain modifications at the bridging ring oxygen, such as CH₂ and S. Nucleotide sugar analogs can also have sugar mimetics, such as cyclobutyl moieties in place of the pentofuranosyl sugar.

Nucleotide analogs can also be modified at the phosphate moiety. Modified phosphate moieties include, but are not limited to, those that can be modified so that the linkage between two nucleotides contains a phosphorothioate, chiral phosphorothioate, phosphorodithioate, phosphotriester, aminoalkylphosphotriester, methyl and other alkyl phosphonates including 3'-alkylene phosphonate and chiral phosphonates, phosphinates, phosphoramidates including 3'-amino phosphoramidate and aminoalkylphosphoramidates, thionophosphoramidates, thionoalkylphosphonates, thionoalkylphosphotriesters, and boranophosphates. These phosphate or modified phosphate linkage between two nucleotides can be through a 3'-5' linkage or a 2'-5' linkage, and the linkage can contain inverted polarity such as 3'-5' to 5'-3' or 2'-5' to 5'-2'. Various salts, mixed salts, and free acid forms are also included. Nucleotide substitutes also include peptide nucleic acids (PNAs).

The present disclosure also provides vectors comprising any one or more of the nucleic acid molecules disclosed herein. In some embodiments, the vectors comprise any one or more of the nucleic acid molecules disclosed herein and a heterologous nucleic acid. The vectors can be viral or nonviral vectors capable of transporting a nucleic acid molecule. In some embodiments, the vector is a plasmid or cosmid (such as, for example, a circular doublestranded DNA into which additional DNA segments can be ligated). In some embodiments, the vector is a viral vector, wherein additional DNA segments can be ligated into the viral genome. Expression vectors include, but are not limited to, plasmids, cosmids, retroviruses, adenoviruses, adeno-associated viruses (AAV), plant viruses such as cauliflower mosaic virus and tobacco mosaic virus, yeast artificial chromosomes (YACs), Epstein-Barr (EBV)-derived episomes, and other expression vectors known in the art.

Desired regulatory sequences for mammalian host cell expression can include, for example, viral elements that direct high levels of polypeptide expression in mammalian cells, such as promoters and/or enhancers derived from retroviral LTRs, cytomegalovirus (CMV) (such as, for example, CMV promoter/enhancer), Simian Virus 40 (SV40) (such as, for example, SV40 promoter/enhancer), adenovirus, (such as, for example, the adenovirus major late promoter (AdMLP)), polyoma and strong mammalian promoters such as native immunoglobulin and actin promoters. Methods of expressing polypeptides in bacterial cells or fungal cells (such as, for example, yeast cells) are also well known. A promoter can be, for example, a constitutively active promoter, a conditional promoter, an inducible promoter, a temporally restricted promoter (such as, for example, a developmentally regulated promoter), or a spatially restricted promoter (such as, for example, a cell-specific or tissue-specific promoter).

Percent identity (or percent complementarity) between particular stretches of nucleotide sequences within nucleic acid molecules or amino acid sequences within polypeptides can be determined routinely using BLAST programs (basic local alignment search tools) and PowerBLAST programs (Altschul et al., J. Mol. Biol., 1990, 215, 403-410; Zhang and Madden, Genome Res., 1997, 7, 649-656) or by using the Gap program (Wisconsin Sequence Analysis Package, Version 8 for Unix, Genetics Computer Group, University Research Park, Madison Wis.), using default settings, which uses the algorithm of Smith and Waterman (Adv. Appl. Math., 1981, 2, 482-489). Herein, if reference is made to percent sequence identity, the higher percentages of sequence identity are preferred over the lower ones.

The present disclosure also provides compositions comprising any one or more of the isolated nucleic acid molecules, genomic nucleic acid molecules, mRNA molecules, and/or cDNA molecules disclosed herein. In some embodiments, the composition is a pharmaceutical composition. In some embodiments, the compositions comprise a carrier and/or excipient. Examples of carriers include, but are not limited to, poly(lactic acid) (PLA) microspheres, poly(D,L-lactic-coglycolic-acid) (PLGA) microspheres, liposomes, micelles, inverse micelles, lipid cochleates, and lipid microtubules. A carrier may comprise a buffered salt solution such as PBS, HBSS, etc.

As used herein, the phrase "corresponding to" or grammatical variations thereof when used in the context of the numbering of a particular nucleotide or nucleotide sequence or position refers to the numbering of a specified reference sequence when the particular nucleotide or nucleotide sequence is compared to a reference sequence (such as, for example, SEQ ID NO:1, SEQ ID NO:3, or SEQ ID NO:9). In other words, the residue (such as, for example, nucleotide or amino acid) number or residue (such as, for example, nucleotide or amino acid) position of a particular polymer is designated with respect to the reference sequence rather than by the actual numerical position of the residue within the particular nucleotide or nucleotide sequence. For example, a particular nucleotide sequence can be aligned to a reference sequence by introducing gaps to optimize residue matches between the two sequences. In these cases, although the gaps are present, the numbering of the residue in the particular nucleotide or nucleotide sequence is made with respect to the reference sequence to which it has been aligned.

For example, a nucleic acid molecule comprising a nucleotide sequence encoding a human FSCN2 polypeptide, wherein the nucleotide sequence comprises a thymine at a position corresponding to position 548 according to SEQ ID NO:2 means that if the nucleotide sequence of the FSCN2 genomic nucleic acid molecule is aligned to the sequence of SEQ ID NO:2, the FSCN2 sequence has a thymine residue at the position that corresponds to position 548 of SEQ ID NO:2. The same applies for mRNA molecules comprising a nucleotide sequence encoding a human FSCN2 polypeptide, wherein the nucleotide sequence comprises a uracil at a position corresponding to position 548 according to SEQ ID NO:6, and cDNA molecules comprising a nucleotide sequence encoding a human FSCN2 polypeptide, wherein the nucleotide sequence comprises a thymine at a position corresponding to position 548 according to SEQ ID NO:12. In other words, these phrases refer to a nucleic acid molecule encoding an FSCN2 polypeptide, wherein the genomic nucleic acid molecule has a nucleotide sequence that comprises a thymine residue that is homologous to the thymine residue at position 548 of SEQ ID NO:2 (or wherein the mRNA molecule has a nucleotide sequence that comprises a uracil residue that is homologous to the uracil residue at position 548 of SEQ ID NO:6, or wherein the cDNA molecule has a nucleotide sequence that comprises a thymine residue that is homologous to the thymine residue at position 548 of SEQ ID NO:12). Herein, such a sequence is also referred to as "FSCN2 sequence with the H138Y "Long" alteration" or "FSCN2 sequence with the H138Y "Long" variation" referring to genomic nucleic acid molecules (or "FSCN2 sequence with the C548U alteration" or "FSCN2 sequence with the C548U variation" referring to mRNA molecules, and "FSCN2 sequence with the C548T alteration" or "FSCN2 sequence with the C548T variation" referring to cDNA molecules).

As described herein, a position within an FSCN2 genomic nucleic acid molecule that corresponds to position 548 according to SEQ ID NO:2, for example, can be identified by performing a sequence alignment between the nucleotide sequence of a particular FSCN2 nucleic acid molecule and the nucleotide sequence of SEQ ID NO:2. A variety of computational algorithms exist that can be used for performing a sequence alignment to identify a nucleotide position that corresponds to, for example, position 548 in SEQ ID NO:2. For example, by using the NCBI BLAST algorithm (Altschul et al., Nucleic Acids Res., 1997, 25, 3389-3402) or CLUSTALW software (Sievers and Higgins, Methods Mol. Biol., 2014, 1079, 105-116) sequence alignments may be performed. However, sequences can also be aligned manually.

The amino acid sequence of an FSCN2 reference polypeptide is set forth in SEQ ID NO:15. Referring to SEQ ID NO:15, the FSCN2 reference polypeptide is 492 amino acids in length. Referring to SEQ ID NO:15, position 138 is histidine.

The amino acid sequence of another FSCN2 reference polypeptide is set forth in SEQ ID NO:16. Referring to SEQ ID NO:16, the FSCN2 reference polypeptide is 516 amino acids in length. Referring to SEQ ID NO:16, position 138 is histidine.

AN FSCN2 variant polypeptide exists (H138Y Short or His138Tyr Short), the amino acid sequence of which is set forth in SEQ ID NO:17. Referring to SEQ ID NO:17, the FSCN2 variant polypeptide is 492 amino acids in length. Referring to SEQ ID NO:17, position 138 is tyrosine.

Another FSCN2 variant polypeptide exists (H138Y Long or His138Tyr Long), the amino acid sequence of which is set forth in SEQ ID NO:18. Referring to SEQ ID NO:18, the FSCN2 variant polypeptide is 516 amino acids in length. Referring to SEQ ID NO:18, position 138 is tyrosine.

The nucleotide and amino acid sequences listed in the accompanying sequence listing are shown using standard letter abbreviations for nucleotide bases, and three-letter code for amino acids. The nucleotide sequences follow the standard convention of beginning at the 5' end of the sequence and proceeding forward (i.e., from left to right in each line) to the 3' end. Only one strand of each nucleotide sequence is shown, but the complementary strand is understood to be included by any reference to the displayed strand. The amino acid sequence follows the standard convention of beginning at the amino terminus of the sequence and proceeding forward (i.e., from left to right in each line) to the carboxy terminus.

The present disclosure also provides therapeutic agents that treat or inhibit hearing loss for use in the treatment of hearing loss (or for use in the preparation of a medicament for treating hearing loss) in a subject, wherein the subject has any of the variant genomic nucleic acid molecules, mRNA molecules, and/or cDNA molecules encoding a human FSCN2 variant polypeptide described herein. The therapeutic agents that treat or inhibit hearing loss can be any of the therapeutic agents that treat or inhibit hearing loss described herein.

In some embodiments, the subject comprises: a genomic nucleic acid molecule having a nucleotide sequence encoding a human FSCN2 polypeptide, wherein the nucleotide sequence comprises a thymine at a position corresponding to position 548 according to SEQ ID NO:2, or the complement thereof; an mRNA molecule having a nucleotide sequence encoding a human FSCN2 polypeptide, wherein the nucleotide sequence comprises a uracil at a position corresponding to position 548 according to SEQ ID NO:6, or the complement thereof; a cDNA molecule having a nucleotide sequence encoding a human FSCN2 polypeptide, wherein the nucleotide sequence comprises a thymine at a position corresponding to position 548 according to SEQ ID NO:12, or the complement thereof; or an FSCN2 polypeptide that comprises a tyrosine at a position corresponding to position 138 according to SEQ ID NO:17.

In some embodiments, the subject comprises: an mRNA molecule having a nucleotide sequence encoding a human FSCN2 polypeptide, wherein the nucleotide sequence comprises a uracil at a position corresponding to position 469 according to SEQ ID NO:7, or the complement thereof; a cDNA molecule having a nucleotide sequence encoding a human FSCN2 polypeptide, wherein the nucleotide sequence comprises a thymine at a position corresponding to position 469 according to SEQ ID NO:13, or the complement thereof; or an FSCN2 polypeptide that comprises a tyrosine at a position corresponding to position 138 according to SEQ ID NO:18.

In some embodiments, the subject comprises an mRNA molecule having a nucleotide sequence encoding a human FSCN2 polypeptide, wherein the nucleotide sequence comprises a uracil at a position corresponding to position 553 according to SEQ ID NO:8, or the complement thereof or a cDNA molecule having a nucleotide sequence encoding a human FSCN2 polypeptide, wherein the nucleotide sequence comprises a thymine at a position corresponding to position 553 according to SEQ ID NO:14, or the complement thereof.

In some embodiments, the subject comprises an mRNA molecule having a nucleotide sequence encoding a human FSCN2 polypeptide, wherein the nucleotide sequence comprises a uracil at a position corresponding to position 567 according to SEQ ID NO:20, or the complement thereof or a cDNA molecule having a nucleotide sequence encoding a human FSCN2 polypeptide, wherein the nucleotide sequence comprises a thymine at a position corresponding to position 567 according to SEQ ID NO:22, or the complement thereof.

The following examples are provided to describe the embodiments in greater detail. They are intended to illustrate, not to limit, the claimed embodiments. The following examples provide those of ordinary skill in the art with a disclosure and description of how the compounds, compositions, articles, devices and/or methods described herein are made and evaluated, and are intended to be purely exemplary and are not intended to limit the scope of any claims. Efforts have been made to ensure accuracy with respect to numbers (such as, for example, amounts, temperature, etc.), but some errors and deviations may be accounted for. Unless indicated otherwise, parts are parts by weight, temperature is in °C or is at ambient temperature, and pressure is at or near atmospheric.

### Examples

### Example 1: FSCN2 Missence Variant is Associated with Hearing Loss

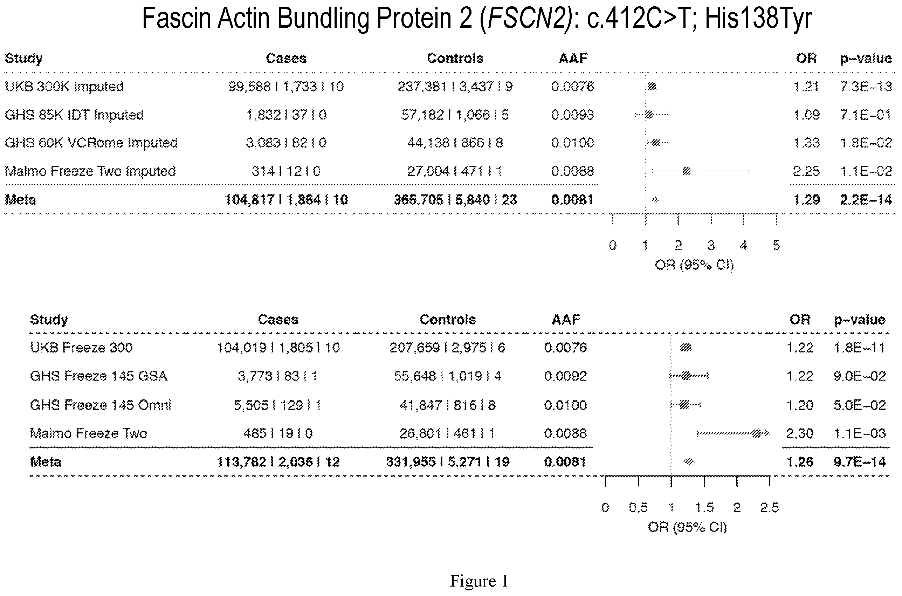

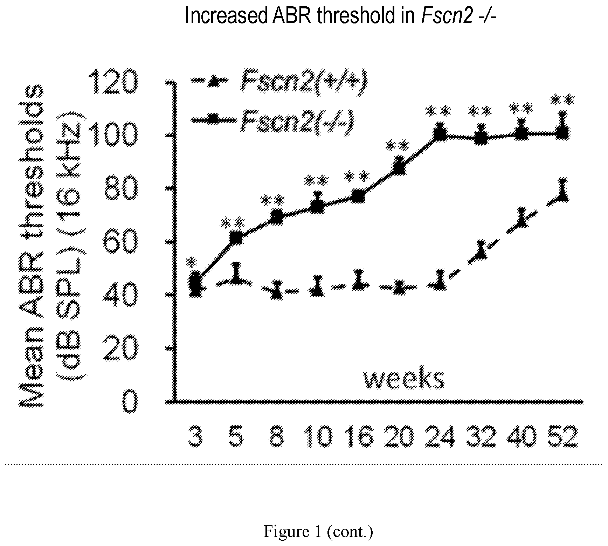

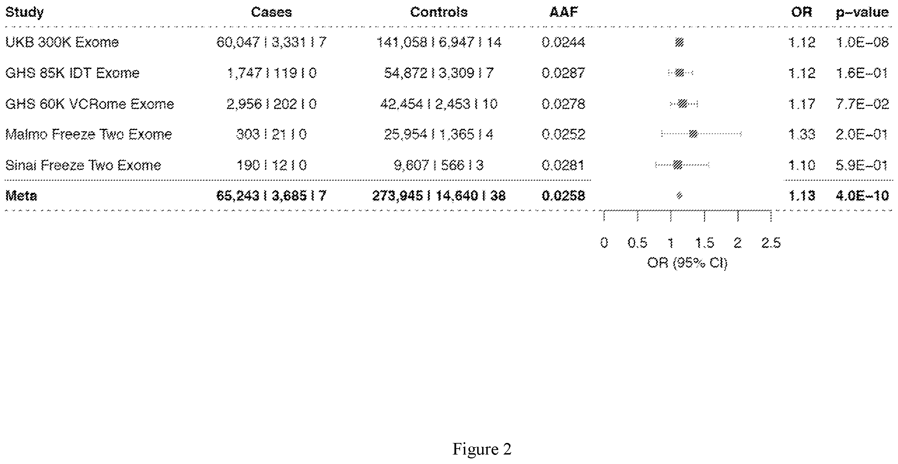

A genome-wide and exome-wide analysis of risk of hearing loss was carried out in UK Biobank and other datasets. A missense variant in *FSCN2* was associated with increased risk of hearing loss (OR = 1.29, p = 2.2E-14) in UK Biobank (see, Figure 1). An association of hearing loss with an aggregate of predicted loss of function and rare (minor allele frequency less than 1%) missense variants in *FSCN2* was also observed in gene burdent tests suggesting that several variants in *FSCN2* may increase the risk of hearing loss in carriers (see, Figure 2). The variants included in Figure 2 include: Variant 17:81528943:C:T (hgvsc = c.412C>T:c.412C>T; hgvsp = p.His138Tyr:p.His138Tyr), Variant 17:81528544:G:A (hgvsc = c.13G>A:c.13G>A; hgvsp = p.Gly5Ser:p.Gly5Ser), Variant 17:81529127:G:A (hgvsc = c.596G>A:c.596G>A; hgvsp = p.Arg199His:p.Arg199His), Variant 17:81536262:T:C (hgvsc = c.1100T>C:c.1100T>C; hgvsp = p.Phe367Ser:p.Phe367Ser), Variant 17:81529204:G:A (hgvsc = c.673G>A:c.673G>A; hgvsp = p.Asp225Asn:p.Asp225Asn), Variant 17:81529336:C:T (hgvsc = c.805C>T:c.805C>T; hgvsp = p.Arg269Cys:p.Arg269Cys), Variant 17:81528998:C:T (hgvsc = c.467C>T:c.467C>T; hgvsp = p.Pro156Leu:p.Pro156Leu), Variant 17:81529190:C:A (hgvsc = c.659C>A:c.659C>A; hgvsp = p.Ala220Asp:p.Ala220Asp), Variant 17:81529083:C:G (hgvsc = c.552C>G:c.552C>G; hgvsp = p.Tyr184*:p.Tyr184*), Variant 17:81529342:G:A (hgvsc = c.811G>A:c.811G>A; hgvsp = p.Val271Ile:p.Val271Ile), Variant 17:81536966:G:C (hgvsc = c.1365G>C:c.1437G>C; hgvsp = p.Glu455Asp:p.Glu479Asp), and Variant 17:81528599:A:G (hgvsc = c.68A>G:c.68A>G; hgvsp = p. Tyr23Cys: p. Tyr23Cys).

## Claims

1. A method of identifying a subject having an increased risk for developing hearing loss, wherein the method comprises:
determining the presence or absence of an Fascin-2 (FSCN2) predicted loss-of-function variant nucleic acid molecule encoding a human FSCN2 polypeptide in a biological sample obtained from the subject, wherein the encoded polypeptide comprises H138Y;
wherein:
when the subject is FSCN2 reference, then the subject does not have an increased risk for developing hearing loss, wherein a subject is FSCN2 reference if the subject lacks an FSCN2 predicted loss-of-function variant nucleic acid molecule; and
when the subject is heterozygous for an FSCN2 predicted loss-of-function variant or homozygous for an FSCN2 predicted loss-of-function variant, then the subject has an increased risk for developing hearing loss.

2. The method according to claim 1, wherein the FSCN2 predicted loss-of-function variant nucleic acid molecule is:
a genomic nucleic acid molecule having a nucleotide sequence comprising a thymine at a position corresponding to position 548 according to SEQ ID NO:2;
an mRNA molecule having a nucleotide sequence comprising: a uracil at a position corresponding to position 548 according to SEQ ID NO:6, a uracil at a position corresponding to position 469 according to SEQ ID NO:7, a uracil at a position corresponding to position 553 according to SEQ ID NO:8, or a uracil at a position corresponding to position 567 according to SEQ ID NO:20; or
a cDNA molecule produced from an mRNA molecule, wherein the cDNA molecule has a nucleotide sequence comprising: a thymine at a position corresponding to position 548 according to SEQ ID NO:12, a thymine at a position corresponding to position 469 according to SEQ ID NO:13, a thymine at a position corresponding to position 553 according to SEQ ID NO:14, or a thymine at a position corresponding to position 567 according to SEQ ID NO:22.

3. The method according to claim 1 or 2, wherein the determining step comprises sequencing at least a portion of the nucleotide sequence of the FSCN2 genomic nucleic acid molecule in the biological sample, wherein the sequenced portion comprises a position corresponding to position 548 according to SEQ ID NO:2, or the complement thereof;
wherein when the sequenced portion of the FSCN2 genomic nucleic acid molecule in the biological sample comprises a thymine at a position corresponding to position 548 according to SEQ ID NO:2, then the FSCN2 genomic nucleic acid molecule in the biological sample is an FSCN2 predicted loss-of-function variant genomic nucleic acid molecule.

4. The method according to claim 1 or 2, wherein the determining step comprises sequencing at least a portion of the nucleotide sequence of the FSCN2 mRNA molecule in the biological sample, wherein the sequenced portion comprises a position corresponding to: position 548 according to SEQ ID NO:6, or the complement thereof; position 469 according to SEQ ID NO:7, or the complement thereof; position 553 according to SEQ ID NO:8, or the complement thereof, or position 567 according to SEQ ID NO:20, or the complement thereof;
wherein when the sequenced portion of the FSCN2 mRNA molecule in the biological sample comprises: a uracil at a position corresponding to position 548 according to SEQ ID NO:6, a uracil at a position corresponding to position 469 according to SEQ ID NO:7, a uracil at a position corresponding to position 553 according to SEQ ID NO:8, or a uracil at a position corresponding to position 567 according to SEQ ID NO:20, then the FSCN2 mRNA molecule in the biological sample is an FSCN2 predicted loss-of-function variant mRNA molecule.

5. The method according to claim 1 or 2, wherein the determining step comprises sequencing at least a portion of the nucleotide sequence of the FSCN2 cDNA molecule, wherein the sequenced portion comprises a position corresponding to: position 548 according to SEQ ID NO:12, or the complement thereof; position 469 according to SEQ ID NO:13, or the complement thereof; position 553 according to SEQ ID NO:14, or the complement thereof; or position 567 according to SEQ ID NO:22, or the complement thereof;
wherein when the sequenced portion of the FSCN2 cDNA molecule in the biological sample comprises: a thymine at a position corresponding to position 548 according to SEQ ID NO:12, a thymine at a position corresponding to position 469 according to SEQ ID NO:13, a thymine at a position corresponding to position 553 according to SEQ ID NO:14, or a thymine at a position corresponding to position 567 according to SEQ ID NO:22, then the FSCN2 cDNA molecule in the biological sample is an FSCN2 predicted loss-of-function variant cDNA molecule.

6. The method according to claim 1 or 2, wherein the determining step comprises:
a) contacting the biological sample with a primer hybridizing to a portion of the nucleotide sequence of the FSCN2 genomic nucleic acid molecule that is proximate to a position corresponding to position 548 according to SEQ ID NO:2;
b) extending the primer at least through the position of the nucleotide sequence of the FSCN2 genomic nucleic acid molecule corresponding to position 548 according to SEQ ID NO:2; and
c) determining whether the extension product of the primer comprises a thymine at a position corresponding to position 548 according to SEQ ID NO:2.

7. The method according to claim 1 or 2, wherein the determining step comprises:
a) contacting the biological sample with a primer hybridizing to a portion of the nucleotide sequence of the FSCN2 mRNA molecule that is proximate to a position corresponding to: position 548 according to SEQ ID NO:6, position 469 according to SEQ ID NO:7, position 553 according to SEQ ID NO:8, or position 567 according to SEQ ID NO:20;
b) extending the primer at least through the position of the nucleotide sequence of the FSCN2 mRNA molecule corresponding to: position 548 according to SEQ ID NO:6, position 469 according to SEQ ID NO:7, position 553 according to SEQ ID NO:8, or position 567 according to SEQ ID NO:20; and
c) determining whether the extension product of the primer comprises: a uracil at a position corresponding to position 548 according to SEQ ID NO:6, a uracil at a position corresponding to position 469 according to SEQ ID NO:7, a uracil at a position corresponding to position 553 according to SEQ ID NO:8, or a uracil at a position corresponding to position 567 according to SEQ ID NO:20.

8. The method according to claim 1 or 2, wherein the determining step comprises:
a) contacting the biological sample with a primer hybridizing to a portion of the nucleotide sequence of the FSCN2 cDNA molecule that is proximate to a position corresponding to: position 548 according to SEQ ID NO:12, position 469 according to SEQ ID NO:13, position 553 according to SEQ ID NO:14, or position 567 according to SEQ ID NO:22;
b) extending the primer at least through the position of the nucleotide sequence of the FSCN2 cDNA molecule corresponding to: position 548 according to SEQ ID NO:12, position 469 according to SEQ ID NO:13, position 553 according to SEQ ID NO:14, or position 567 according to SEQ ID NO:22; and
c) determining whether the extension product of the primer comprises: a thymine at a position corresponding to position 548 according to SEQ ID NO:12, a thymine at a position corresponding to position 469 according to SEQ ID NO:13, a thymine at a position corresponding to position 553 according to SEQ ID NO:14, or a thymine at a position corresponding to position 567 according to SEQ ID NO:22.

9. The method according to claim 1 or 2, wherein the determining step comprises:
a) amplifying at least a portion of the nucleic acid molecule that encodes the human FSCN2 polypeptide, wherein the portion comprises a thymine at a position corresponding to position 548 according to SEQ ID NO:2, or the complement thereof;
b) labeling the amplified nucleic acid molecule with a detectable label;
c) contacting the labeled nucleic acid molecule with a support comprising an alteration-specific probe, wherein the alteration-specific probe comprises a nucleotide sequence which hybridizes under stringent conditions to the nucleic acid sequence of the amplified nucleic acid molecule comprising a thymine at a position corresponding to position 548 according to SEQ ID NO:2, or the complement thereof; and
d) detecting the detectable label.

10. The method according to claim 1 or 2, wherein the determining step comprises:
a) amplifying at least a portion of the nucleic acid molecule that encodes the human FSCN2 polypeptide, wherein the portion comprises: a uracil at a position corresponding to position 548 according to SEQ ID NO:6, or the complement thereof; a uracil at a position corresponding to position 469 according to SEQ ID NO:7, or the complement thereof; a uracil at a position corresponding to position 553 according to SEQ ID NO:8, or the complement thereof; or a uracil at a position corresponding to position 567 according to SEQ ID NO:20, or the complement thereof;
b) labeling the amplified nucleic acid molecule with a detectable label;
c) contacting the labeled nucleic acid molecule with a support comprising an alteration-specific probe, wherein the alteration-specific probe comprises a nucleotide sequence which hybridizes under stringent conditions to the nucleic acid sequence of the amplified nucleic acid molecule comprising: a uracil at a position corresponding to position 548 according to SEQ ID NO:6, or the complement thereof; a uracil at a position corresponding to position 469 according to SEQ ID NO:7, or the complement thereof; a uracil at a position corresponding to position 553 according to SEQ ID NO:8, or the complement thereof; or a uracil at a position corresponding to position 567 according to SEQ ID NO:20, or the complement thereof; and
d) detecting the detectable label.

11. The method according to claim 1 or 2, wherein the determining step comprises:
a) amplifying at least a portion of the nucleic acid molecule that encodes the human FSCN2 polypeptide, wherein the portion comprises: a thymine at a position corresponding to position 548 according to SEQ ID NO:12, or the complement thereof; a thymine at a position corresponding to position 469 according to SEQ ID NO:13, or the complement thereof; a thymine at a position corresponding to position 553 according to SEQ ID NO:14, or the complement thereof; or a thymine at a position corresponding to position 567 according to SEQ ID NO:22, or the complement thereof;
b) labeling the amplified nucleic acid molecule with a detectable label;
c) contacting the labeled nucleic acid molecule with a support comprising an alteration-specific probe, wherein the alteration-specific probe comprises a nucleotide sequence which hybridizes under stringent conditions to the nucleic acid sequence of the amplified nucleic acid molecule comprising: a thymine at a position corresponding to position 548 according to SEQ ID NO:12, or the complement thereof; a thymine at a position corresponding to position 469 according to SEQ ID NO:13, or the complement thereof; a thymine at a position corresponding to position 553 according to SEQ ID NO:14, or the complement thereof; or a thymine at a position corresponding to position 567 according to SEQ ID NO:22, or the complement thereof; and
d) detecting the detectable label.

12. A therapeutic agent that treats or inhibits hearing loss for use in the treatment of hearing loss in a subject having:
a genomic nucleic acid molecule having a nucleotide sequence encoding a human Fascin-2 (FSCN2) polypeptide, wherein the nucleotide sequence comprises a thymine at a position corresponding to position 548 according to SEQ ID NO:2, or the complement thereof;
an mRNA molecule having a nucleotide sequence encoding a human FSCN2 polypeptide, wherein the nucleotide sequence comprises: a uracil at a position corresponding to position 548 according to SEQ ID NO:6, or the complement thereof; a uracil at a position corresponding to position 469 according to SEQ ID NO:7, or the complement thereof; a uracil at a position corresponding to position 553 according to SEQ ID NO:8, or the complement thereof; or a uracil at a position corresponding to position 567 according to SEQ ID NO:20, or the complement thereof; or
a cDNA molecule having a nucleotide sequence encoding a human FSCN2 polypeptide, wherein the nucleotide sequence comprises: a thymine at a position corresponding to position 548 according to SEQ ID NO:12, or the complement thereof; a thymine at a position corresponding to position 469 according to SEQ ID NO:13, or the complement thereof; a thymine at a position corresponding to position 553 according to SEQ ID NO:14, or the complement thereof; or a thymine at a position corresponding to position 567 according to SEQ ID NO:22, or the complement thereof,
wherein the therapeutic agent comprises an antioxidant, a calcium-channel blocker, an antiinflammatory drug, a steroid, an apoptosis inhibitor, D-methionine, ebselen, N-acetylcysteine, lipoic acid, the combination of ebselen and allopurinol, resveratrol, a neurotrophic factor, T-817MA, a caspase inhibitor, z-DEVD-fmk, a copper transport inhibitor, cimetidine and copper sulphate, or a micronutrient with antioxidant vitamin.

## Patentansprüche

1. Verfahren zur Identifizierung eines Individuums, das ein erhöhtes Risiko aufweist, einen Hörverlust zu entwickeln, wobei das Verfahren umfasst:
Bestimmen des Vorhandenseins oder Nichtvorhandenseins eines Fascin-2-(FSCN2)-vorhergesagte Funktionsverlust-Varianten-Nukleinsäuremoleküls, das ein humanes FSCN2-Polypeptid kodiert, in einer biologischen Probe, die von dem Individuum erhalten wurde, wobei das kodierte Polypeptid H138Y umfasst;
wobei:
wenn das Individuum FSCN2-Referenz ist, dann weist das Individuum kein erhöhtes Risiko auf, einen Hörverlust zu entwickeln, wobei ein Individuum FSCN2-Referenz ist, wenn dem Individuum ein FSCN2-vorhergesagte Funktionsverlust-Varianten-Nukleinsäuremolekül fehlt; und
wenn das Individuum heterozygot für eine FSCN2-vorhergesagte Funktionsverlust-Variante oder homozygot für eine FSCN2-vorhergesagte Funktionsverlust-Variante ist, dann weist das Individuum ein erhöhtes Risiko für die Entwicklung von Hörverlust auf.

2. Verfahren nach Anspruch 1, wobei das FSCN2-vorhergesagte Funktionsverlust-Varianten-Nukleinsäuremolekül ist:
ein genomisches Nukleinsäuremolekül, das eine Nukleotidsequenz aufweist, die ein Thymin an einer Position umfasst entsprechend Position 548 gemäß SEQ ID NO:2;
ein mRNA-Molekül, das eine Nukleotidsequenz aufweist, die umfasst: ein Uracil an einer Position entsprechend Position 548 gemäß SEQ ID NO:6, ein Uracil an einer Position entsprechend Position 469 gemäß SEQ ID NO:7, ein Uracil an einer Position entsprechend Position 553 gemäß SEQ ID NO:8, oder ein Uracil an einer Position entsprechend Position 567 gemäß SEQ ID NO:20; oder
ein cDNA-Molekül, das aus einem mRNA-Molekül hergestellt wird, wobei das cDNA-Molekül eine Nukleotidsequenz aufweist, die umfasst: ein Thymin an einer Position entsprechend Position 548 gemäß SEQ ID NO:12, ein Thymin an einer Position entsprechend Position 469 gemäß SEQ ID NO:13, ein Thymin an einer Position entsprechend Position 553 gemäß SEQ ID NO:14, oder ein Thymin an einer Position entsprechend Position 567 gemäß SEQ ID NO:22.

3. Verfahren nach Anspruch 1 oder 2, wobei der Bestimmungsschritt das Sequenzieren mindestens eines Teils der Nukleotidsequenz des genomischen FSCN2-Nukleinsäuremoleküls in der biologischen Probe umfasst, wobei der sequenzierte Teil eine Position entsprechend Position 548 gemäß SEQ ID NO:2 oder deren Komplement umfasst;
wobei, wenn der sequenzierte Teil des genomischen FSCN2-Nukleinsäuremoleküls in der biologischen Probe ein Thymin an einer Position entsprechend Position 548 gemäß SEQ ID NO:2 umfasst, das genomische FSCN2-Nukleinsäuremolekül in der biologischen Probe dann ein genomisches FSCN2-vorhergesagte Funktionsverlust-Varianten-Nukleinsäuremolekül ist.

4. Verfahren nach Anspruch 1 oder 2, wobei der Bestimmungsschritt das Sequenzieren mindestens eines Teils der Nukleotidsequenz des FSCN2-mRNA-Moleküls in der biologischen Probe umfasst, wobei der sequenzierte Teil eine Position entsprechend: Position 548 gemäß SEQ ID NO:6 oder deren Komplement; Position 469 gemäß SEQ ID NO:7 oder deren Komplement; Position 553 gemäß SEQ ID NO:8 oder deren Komplement, oder Position 567 gemäß SEQ ID NO:20 oder deren Komplement, umfasst;
wobei, wenn der sequenzierte Teil des FSCN2-mRNA-Moleküls in der biologischen Probe umfasst: ein Uracil an einer Position entsprechend Position 548 gemäß SEQ ID NO:6, ein Uracil an einer Position entsprechend Position 469 gemäß SEQ ID NO:7, ein Uracil an einer Position entsprechend Position 553 gemäß SEQ ID NO:8, oder ein Uracil an einer Position entsprechend Position 567 gemäß SEQ ID NO:20, das FSCN2-mRNA-Molekül in der biologischen Probe dann ein FSCN2-vorhergesagte Funktionsverlust-Varianten-mRNA-Molekül ist.

5. Verfahren nach Anspruch 1 oder 2, wobei der Bestimmungsschritt das Sequenzieren mindestens eines Teils der Nukleotidsequenz des FSCN2-cDNA-Moleküls umfasst, wobei der sequenzierte Teil eine Position entsprechend: Position 548 gemäß SEQ ID NO:12 oder deren Komplement; Position 469 gemäß SEQ ID NO:13 oder deren Komplement; Position 553 gemäß SEQ ID NO:14 oder deren Komplement; oder Position 567 gemäß SEQ ID NO:22 oder deren Komplement, umfasst;
wobei, wenn der sequenzierte Teil des FSCN2-cDNA-Moleküls in der biologischen Probe umfasst: ein Thymin an einer Position entsprechend Position 548 gemäß SEQ ID NO:12, ein Thymin an einer Position entsprechend Position 469 gemäß SEQ ID NO:13, ein Thymin an einer Position entsprechend Position 553 gemäß SEQ ID NO:14, oder ein Thymin an einer Position entsprechend Position 567 gemäß SEQ ID NO:22, das FSCN2-cDNA-Molekül in der biologischen Probe dann ein FSCN2-vorhergesagte Funktionsverlust-Varianten-cDNA-Molekül ist.

6. Verfahren nach Anspruch 1 oder 2, wobei der Bestimmungsschritt umfasst:
a) Inkontaktbringen der biologischen Probe mit einem Primer, der an einen Teil der Nukleotidsequenz des genomischen FSCN2-Nukleinsäuremoleküls hybridisiert, der nahe einer Position entsprechend Position 548 gemäß SEQ ID NO:2 liegt;
b) Verlängern des Primers mindestens durch die Position der Nukleotidsequenz des genomischen FSCN2-Nukleinsäuremoleküls entsprechend Position 548 gemäß SEQ ID NO:2; und
c) Bestimmen, ob das Verlängerungsprodukt des Primers ein Thymin an einer Position entsprechend Position 548 gemäß SEQ ID NO:2 umfasst.

7. Verfahren nach Anspruch 1 oder 2, wobei der Bestimmungsschritt umfasst:
a) Inkontaktbringen der biologischen Probe mit einem Primer, der an einen Teil der Nukleotidsequenz des FSCN2-mRNA-Moleküls hybridisiert, der nahe einer Position liegt entsprechend: Position 548 gemäß SEQ ID NO:6, Position 469 gemäß SEQ ID NO:7, Position 553 gemäß SEQ ID NO:8, oder Position 567 gemäß SEQ ID NO:20;
b) Verlängern des Primers mindestens durch die Position der Nukleotidsequenz des FSCN2-mRNA-Moleküls entsprechend: Position 548 gemäß SEQ ID NO:6, Position 469 gemäß SEQ ID NO:7, Position 553 gemäß SEQ ID NO:8, oder Position 567 gemäß SEQ ID NO:20; und
c) Bestimmen, ob das Verlängerungsprodukt des Primers umfasst: ein Uracil an einer Position entsprechend Position 548 gemäß SEQ ID NO:6, ein Uracil an einer Position entsprechend Position 469 gemäß SEQ ID NO:7, ein Uracil an einer Position entsprechend Position 553 gemäß SEQ ID NO:8, oder ein Uracil an einer Position entsprechend Position 567 gemäß SEQ ID NO:20.

8. Verfahren nach Anspruch 1 oder 2, wobei der Bestimmungsschritt umfasst:
a) Inkontaktbringen der biologischen Probe mit einem Primer, der an einen Teil der Nukleotidsequenz des FSCN2-cDNA-Moleküls hybridisiert, der nahe einer Position liegt entsprechend: Position 548 gemäß SEQ ID NO:12, Position 469 gemäß SEQ ID NO:13, Position 553 gemäß SEQ ID NO:14, oder Position 567 gemäß SEQ ID NO:22;
b) Verlängern des Primers mindestens durch die Position der Nukleotidsequenz des FSCN2-cDNA-Moleküls entsprechend: Position 548 gemäß SEQ ID NO:12, Position 469 gemäß SEQ ID NO:13, Position 553 gemäß SEQ ID NO:14, oder Position 567 gemäß SEQ ID NO:22; und
c) Bestimmen, ob das Verlängerungsprodukt des Primers umfasst: ein Thymin an einer Position entsprechend Position 548 gemäß SEQ ID NO:12, ein Thymin an einer Position entsprechend Position 469 gemäß SEQ ID NO:13, ein Thymin an einer Position entsprechend Position 553 gemäß SEQ ID NO:14, oder ein Thymin an einer Position entsprechend Position 567 gemäß SEQ ID NO:22.

9. Verfahren nach Anspruch 1 oder 2, wobei der Bestimmungsschritt umfasst:
a) Amplifizieren mindestens eines Teils des Nukleinsäuremoleküls, das das humane FSCN2-Polypeptid kodiert, wobei der Teil ein Thymin an einer Position entsprechend Position 548 gemäß SEQ ID NO:2 oder deren Komplement umfasst;
b) Markieren des amplifizierten Nukleinsäuremoleküls mit einem nachweisbaren Marker;
c) Inkontaktbringen des markierten Nukleinsäuremoleküls mit einem Träger, der eine änderungsspezifische Sonde umfasst, wobei die änderungsspezifische Sonde eine Nukleotidsequenz umfasst, die unter stringenten Bedingungen an die Nukleinsäuresequenz des amplifizierten Nukleinsäuremoleküls hybridisiert, das ein Thymin an einer Position entsprechend Position 548 gemäß SEQ ID NO:2 oder deren Komplement umfasst; und
d) Nachweisen des nachweisbaren Markers.

10. Verfahren nach Anspruch 1 oder 2, wobei der Bestimmungsschritt umfasst:
a) Amplifizieren mindestens eines Teils des Nukleinsäuremoleküls, das das humane FSCN2-Polypeptid kodiert, wobei der Teil umfasst: ein Uracil an einer Position entsprechend Position 548 gemäß SEQ ID NO:6 oder deren Komplement; ein Uracil an einer Position entsprechend Position 469 gemäß SEQ ID NO:7 oder deren Komplement; ein Uracil an einer Position entsprechend Position 553 gemäß SEQ ID NO:8 oder deren Komplement; oder ein Uracil an einer Position entsprechend Position 567 gemäß SEQ ID NO:20 oder deren Komplement;
b) Markieren des amplifizierten Nukleinsäuremoleküls mit einem nachweisbaren Marker;
c) Inkontaktbringen des markierten Nukleinsäuremoleküls mit einem Träger, der eine änderungsspezifische Sonde umfasst, wobei die änderungsspezifische Sonde eine Nukleotidsequenz umfasst, die unter stringenten Bedingungen an die Nukleinsäuresequenz des amplifizierten Nukleinsäuremoleküls hybridisiert, das umfasst: ein Uracil an einer Position entsprechend Position 548 gemäß SEQ ID NO:6 oder deren Komplement; ein Uracil an einer Position entsprechend Position 469 gemäß SEQ ID NO:7 oder deren Komplement; ein Uracil an einer Position entsprechend Position 553 gemäß SEQ ID NO:8 oder deren Komplement; oder ein Uracil an einer Position entsprechend Position 567 gemäß SEQ ID NO:20 oder deren Komplement; und
d) Nachweisen des nachweisbaren Markers.

11. Verfahren nach Anspruch 1 oder 2, wobei der Bestimmungsschritt umfasst:
a) Amplifizieren mindestens eines Teils des Nukleinsäuremoleküls, das das humane FSCN2-Polypeptid kodiert, wobei der Teil umfasst: ein Thymin an einer Position entsprechend Position 548 gemäß SEQ ID NO:12 oder deren Komplement; ein Thymin an einer Position entsprechend Position 469 gemäß SEQ ID NO:13 oder deren Komplement; ein Thymin an einer Position entsprechend Position 553 gemäß SEQ ID NO:14 oder deren Komplement; oder ein Thymin an einer Position entsprechend Position 567 gemäß SEQ ID NO:22 oder deren Komplement;
b) Markieren des amplifizierten Nukleinsäuremoleküls mit einem nachweisbaren Marker;
c) Inkontaktbringen des markierten Nukleinsäuremoleküls mit einem Träger, der eine änderungsspezifische Sonde umfasst, wobei die änderungsspezifische Sonde eine Nukleotidsequenz umfasst, die unter stringenten Bedingungen an die Nukleinsäuresequenz des amplifizierten Nukleinsäuremoleküls hybridisiert, das umfasst: ein Thymin an einer Position entsprechend Position 548 gemäß SEQ ID NO:12 oder deren Komplement; ein Thymin an einer Position entsprechend Position 469 gemäß SEQ ID NO:13 oder deren Komplement; ein Thymin an einer Position entsprechend Position 553 gemäß SEQ ID NO:14 oder deren Komplement; oder ein Thymin an einer Position entsprechend Position 567 gemäß SEQ ID NO:22 oder deren Komplement; und
d) Nachweisen des nachweisbaren Markers.

12. Therapeutisches Agens, das Hörverlust behandelt oder hemmt, zur Verwendung bei der Behandlung von Hörverlust bei einem Individuum, das aufweist:
ein genomisches Nukleinsäuremolekül, das eine Nukleotidsequenz aufweist, die ein humanes Fascin-2 (FSCN2) Polypeptid kodiert, wobei die Nukleotidsequenz ein Thymin an einer Position entsprechend Position 548 gemäß SEQ ID NO:2 oder deren Komplement umfasst;
ein mRNA-Molekül, das eine Nukleotidsequenz aufweist, die ein humanes FSCN2-Polypeptid kodiert, wobei die Nukleotidsequenz umfasst: ein Uracil an einer Position entsprechend Position 548 gemäß SEQ ID NO:6 oder deren Komplement; ein Uracil an einer Position entsprechend Position 469 gemäß SEQ ID NO:7 oder deren Komplement; ein Uracil an einer Position entsprechend Position 553 gemäß SEQ ID NO:8 oder deren Komplement; oder ein Uracil an einer Position entsprechend Position 567 gemäß SEQ ID NO:20 oder deren Komplement; oder
ein cDNA-Molekül, das eine Nukleotidsequenz aufweist, die ein humanes FSCN2-Polypeptid kodiert, wobei die Nukleotidsequenz umfasst: ein Thymin an einer Position entsprechend Position 548 gemäß SEQ ID NO:12 oder deren Komplement; ein Thymin an einer Position entsprechend Position 469 gemäß SEQ ID NO:13 oder deren Komplement; ein Thymin an einer Position entsprechend Position 553 gemäß SEQ ID NO:14 oder deren Komplement; oder ein Thymin an einer Position entsprechend Position 567 gemäß SEQ ID NO:22 oder deren Komplement,
wobei das therapeutische Agens ein Antioxidans, einen Calciumkanalblocker, ein entzündungshemmendes Medikament, ein Steroid, einen Apoptose-Inhibitor, D-Methionin, Ebselen, N-Acetylcystein, Liponsäure, die Kombination von Ebselen und Allopurinol, Resveratrol, einen neurotrophen Faktor, T-817MA, einen Caspase-Inhibitor, z-DEVD-fmk, einen Kupfertransport-Inhibitor, Cimetidin und Kupfersulfat, oder einen Mikronährstoff mit antioxidativem Vitamin umfasst.

## Revendications

1. Méthode d'identification d'un sujet présentant un risque accru de développer une perte d'audition, dans laquelle la méthode comprend :
la détermination de la présence ou de l'absence d'une molécule d'acide nucléique variant à perte de fonction prédite de Fascine-2 (FSCN2) codant pour un polypeptide de FSCN2 humain dans un échantillon biologique obtenu auprès dudit sujet, où le polypeptide codé comprend H138Y ;
dans laquelle :
lorsque le sujet est la référence de FSCN2, alors le sujet ne présente pas de risque accru de développer une perte d'audition, où un sujet est la référence de FSCN2 si le sujet est dépourvu d'une molécule d'acide nucléique variant à perte de fonction prédite de FSCN2 ; et
lorsque le sujet est hétérozygote pour un variant à perte de fonction prédite de FSCN2 ou homozygote pour un variant à perte de fonction prédite de FSCN2, alors le sujet présente un risque accru de développer une perte d'audition.

2. Méthode selon la revendication 1, dans laquelle la molécule d'acide nucléique variant à perte de fonction prédite de FSCN2 est :
une molécule d'acide nucléique génomique ayant une séquence nucléotidique comprenant une thymine au niveau d'une position correspondant à la position 548 selon la SEQ ID NO:2 ;
une molécule d'ARNm ayant une séquence nucléotidique comprenant : un uracil au niveau d'une position correspondant à la position 548 selon la SEQ ID NO:6, un uracil au niveau d'une position correspondant à la position 469 selon la SEQ ID NO:7, un uracil au niveau d'une position correspondant à la position 553 selon la SEQ ID NO:8, ou un uracil au niveau d'une position correspondant à la position 567 selon la SEQ ID NO:20 ; ou
une molécule d'ADNc produite à partir d'une molécule d'ARNm, où la molécule d'ADNc a une séquence nucléotidique comprenant : une thymine au niveau d'une position correspondant à la position 548 selon la SEQ ID NO:12, une thymine au niveau d'une position correspondant à la position 469 selon la SEQ ID NO:13, une thymine au niveau d'une position correspondant à la position 553 selon la SEQ ID NO:14, ou une thymine au niveau d'une position correspondant à la position 567 selon la SEQ ID NO:22.

3. Méthode selon la revendication 1 ou 2, dans laquelle l'étape de détermination comprend le séquençage au niveau d'au moins une partie de la séquence nucléotidique de la molécule d'acide nucléique génomique de FSCN2 dans l'échantillon biologique, dans laquelle la partie séquencée comprend une position correspondant à la position 548 selon la SEQ ID NO:2, ou son complément ;
dans laquelle, lorsque la partie séquencée de la molécule d'acide nucléique génomique de FSCN2 dans l'échantillon biologique comprend une thymine au niveau d'une position correspondant à la position 548 selon la SEQ ID NO:2, la molécule d'acide nucléique génomique de FSCN2 dans l'échantillon biologique est alors une molécule d'acide nucléique génomique variant à perte de fonction prédite de FSCN2.

4. Méthode selon la revendication 1 ou 2, dans laquelle l'étape de détermination comprend le séquençage au niveau d'au moins une partie de la séquence nucléotidique de la molécule d'ARNm de FSCN2 dans l'échantillon biologique, où la partie séquencée comprend une position correspondant à : la position 548 selon la SEQ ID NO:6, ou son complément ; la position 469 selon la SEQ ID NO:7, ou son complément ; la position 553 selon la SEQ ID NO:8, ou son complément, ou la position 567 selon la SEQ ID NO:20, ou son complément ;
dans laquelle, lorsque la partie séquencée de la molécule d'ARNm de FSCN2 dans l'échantillon biologique comprend : un uracil au niveau d'une position correspondant à la position 548 selon la SEQ ID NO:6, un uracil au niveau d'une position correspondant à la position 469 selon la SEQ ID NO:7, un uracil au niveau d'une position correspondant à la position 553 selon la SEQ ID NO:8, ou un uracil au niveau d'une position correspondant à la position 567 selon la SEQ ID NO:20, la molécule d'ARNm de FSCN2 dans l'échantillon biologique est alors une molécule d'ARNm variant à perte de fonction prédite de FSCN2.

5. Méthode selon la revendication 1 ou 2, dans laquelle l'étape de détermination comprend le séquençage au niveau d'au moins une partie de la séquence nucléotidique de la molécule d'ADNc de FSCN2, dans laquelle la partie séquencée comprend une position correspondant à : la position 548 selon la SEQ ID NO: 12, ou son complément ; la position 469 selon la SEQ ID NO: 13, ou son complément ; la position 553 selon la SEQ ID NO:14, ou son complément ; ou la position 567 selon la SEQ ID NO:22, ou son complément ;
dans laquelle, lorsque la partie séquencée de la molécule d'ADNc de FSCN2 dans l'échantillon biologique comprend : une thymine au niveau d'une position correspondant à la position 548 selon la SEQ ID NO:12, une thymine au niveau d'une position correspondant à la position 469 selon la SEQ ID NO: 13, une thymine au niveau d'une position correspondant à la position 553 selon la SEQ ID NO:14, ou une thymine au niveau d'une position correspondant à la position 567 selon la SEQ ID NO:22, la molécule d'ADNc de FSCN2 dans l'échantillon biologique est alors une molécule d'ADNc variant à perte de fonction prédite de FSCN2.

6. Méthode selon la revendication 1 ou 2, dans laquelle l'étape de détermination comprend :
a) mettre en contact l'échantillon biologique avec une amorce s'hybridant à une partie de la séquence nucléotidique de la molécule d'acide nucléique génomique de FSCN2 qui est à proximité d'une position correspondant à la position 548 selon la SEQ ID NO:2 ;
b) étendre l'amorce au moins par le biais de la position de la séquence nucléotidique de la molécule d'acide nucléique génomique de FSCN2 correspondant à la position 548 selon la SEQ ID NO:2 ; et
c) déterminer si le produit d'extension de l'amorce comprend une thymine au niveau d'une position correspondant à la position 548 selon la SEQ ID NO:2.

7. Méthode selon la revendication 1 ou 2, dans laquelle l'étape de détermination comprend :
a) mettre en contact l'échantillon biologique avec une amorce s'hybridant à une partie de la séquence nucléotidique de la molécule d'ARNm de FSCN2 qui est à proximité d'une position correspondant à : la position 548 selon la SEQ ID NO:6, la position 469 selon la SEQ ID NO:7, la position 553 selon la SEQ ID NO:8, ou la position 567 selon la SEQ ID NO:20 ;
b) étendre l'amorce au moins par le biais de la position de la séquence nucléotidique de la molécule d'ARNm de FSCN2 correspondant à : la position 548 selon la SEQ ID NO:6, la position 469 selon la SEQ ID NO:7, la position 553 selon la SEQ ID NO:8, ou la position 567 selon la SEQ ID NO:20 ; et
c) déterminer si le produit d'extension de l'amorce comprend :un uracil au niveau d'une position correspondant à la position 548 selon la SEQ ID NO:6, un uracil au niveau d'une position correspondant à la position 469 selon la SEQ ID NO:7, un uracil au niveau d'une position correspondant à la position 553 selon la SEQ ID NO:8, ou un uracil au niveau d'une position correspondant à la position 567 selon la SEQ ID NO:20.

8. Méthode selon la revendication 1 ou 2, dans laquelle l'étape de détermination comprend :
a) mettre en contact l'échantillon biologique avec une amorce s'hybridant à une partie de la séquence nucléotidique de la molécule d'ADNc de FSCN2 qui est à proximité d'une position correspondant à : la position 548 selon la SEQ ID NO:12, la position 469 selon la SEQ ID NO:13, la position 553 selon la SEQ ID NO:14, ou la position 567 selon la SEQ ID NO:22 ;
b) étendre l'amorce au moins par le biais de la position de la séquence nucléotidique de la molécule d'ADNc de FSCN2 correspondant à : la position 548 selon la SEQ ID NO:12, la position 469 selon la SEQ ID NO:13, la position 553 selon la SEQ ID NO:14, ou la position 567 selon la SEQ ID NO:22 ; et
c) déterminer si le produit d'extension de l'amorce comprend :une thymine au niveau d'une position correspondant à la position 548 selon la SEQ ID NO:12, une thymine au niveau d'une position correspondant à la position 469 selon la SEQ ID NO:13, une thymine au niveau d'une position correspondant à la position 553 selon la SEQ ID NO:14, ou une thymine au niveau d'une position correspondant à la position 567 selon la SEQ ID NO:22.

9. Méthode selon la revendication 1 ou 2, dans laquelle l'étape de détermination comprend :
a) l'amplification d'au moins une partie de la molécule d'acide nucléique qui code pour le polypeptide de FSCN2 humain, où la partie comprend une thymine au niveau de la position 548 selon la SEQ ID NO:2 ; ou son complément ;
b) le marquage de la molécule d'acide nucléique amplifiée avec un marqueur détectable ;
c) la mise en contact de la molécule d'acide nucléique marquée avec un support comprenant une sonde spécifique à une altération, où la sonde spécifique à une altération comprend une séquence nucléotidique qui s'hybride dans des conditions stringentes à la séquence d'acide nucléique de la molécule d'acide nucléique amplifiée comprenant une thymine au niveau de la position 548 selon la SEQ ID NO:2, ou son complément ; et
d) la détection du marqueur détectable.

10. Méthode selon la revendication 1 ou 2, dans laquelle l'étape de détermination comprend :
a) l'amplification d'au moins une partie de la molécule d'acide nucléique qui code pour le polypeptide de FSCN2 humain, où la partie comprend : un uracil au niveau d'une position correspondant à la position 548 selon la SEQ ID NO:6, ou son complément ; un uracil au niveau d'une position correspondant à la position 469 selon la SEQ ID NO:7, ou son complément ; un uracil au niveau d'une position correspondant à la position 553 selon la SEQ ID NO:8, ou son complément ; ou un uracil au niveau d'une position correspondant à la position 567 selon la SEQ ID NO:20, ou son complément ;
b) le marquage de la molécule d'acide nucléique amplifiée avec un marqueur détectable ;
c) la mise en contact de la molécule d'acide nucléique marquée avec un support comprenant une sonde spécifique à une altération, où la sonde spécifique à une altération comprend une séquence nucléotidique qui s'hybride dans des conditions stringentes à la séquence d'acide nucléique de la molécule d'acide nucléique amplifiée comprenant : un uracil au niveau d'une position correspondant à la position 548 selon la SEQ ID NO:6, ou son complément ; un uracil au niveau d'une position correspondant à la position 469 selon la SEQ ID NO:7, ou son complément ; un uracil au niveau d'une position correspondant à la position 553 selon la SEQ ID NO:8, ou son complément ; ou un uracil au niveau d'une position correspondant à la position 567 selon la SEQ ID NO:20, ou son complément ; et
d) la détection du marqueur détectable.

11. Méthode selon la revendication 1 ou 2, dans laquelle l'étape de détermination comprend :
a) l'amplification d'au moins une partie de la molécule d'acide nucléique qui code pour le polypeptide de FSCN2 humain, où la partie comprend : une thymine au niveau d'une position correspondant à la position 548 selon la SEQ ID NO:12, ou son complément ; une thymine au niveau d'une position correspondant à la position 469 selon la SEQ ID NO:13, ou son complément ; une thymine au niveau d'une position correspondant à la position 553 selon la SEQ ID NO:14, ou son complément ; ou une thymine au niveau d'une position correspondant à la position 567 selon la SEQ ID NO:22, ou son complément ;
b) le marquage de la molécule d'acide nucléique amplifiée avec un marqueur détectable ;
c) la mise en contact de la molécule d'acide nucléique marquée avec un support comprenant une sonde spécifique à une altération, où la sonde spécifique à une altération comprend une séquence nucléotidique qui s'hybride dans des conditions stringentes à la séquence d'acide nucléique de la molécule d'acide nucléique amplifiée comprenant : une thymine au niveau d'une position correspondant à la position 548 selon la SEQ ID NO:12, ou son complément ; une thymine au niveau d'une position correspondant à la position 469 selon la SEQ ID NO:13, ou son complément ; une thymine au niveau d'une position correspondant à la position 553 selon la SEQ ID NO: 14, ou son complément ; ou une thymine au niveau d'une position correspondant à la position 567 selon la SEQ ID NO:22, ou son complément ; et
d) la détection du marqueur détectable.

12. Agent thérapeutique qui traite ou inhibe la perte d'audition pour uneutilisation dans le traitement de la perte d'audition chez un sujet présentant :
une molécule d'acide nucléique génomique ayant une séquence nucléotidique codant pour un polypeptide de Fascine-2 (FSCN2) humain, où la séquence nucléotidique comprend une thymine au niveau d'une position correspondant à la position 548 selon la SEQ ID NO:2, ou son complément ;
une molécule d'ARNm ayant une séquence nucléotidique codant pour un polypeptide de FSCN2 humain, où la séquence nucléotidique comprend : un uracil au niveau d'une position correspondant à la position 548 selon la SEQ ID NO:6, ou son complément ; un uracil au niveau d'une position correspondant à la position 469 selon la SEQ ID NO:7, ou son complément ; un uracil au niveau d'une position correspondant à la position 553 selon la SEQ ID NO:8, ou son complément ; ou un uracil au niveau d'une position correspondant à la position 567 selon la SEQ ID NO:20, ou son complément ; ou
une molécule d'ADNc ayant une séquence nucléotidique codant pour un polypeptide de FSCN2 humain, où la séquence nucléotidique comprend : une thymine au niveau d'une position correspondant à la position 548 selon la SEQ ID NO:12, ou son complément ; une thymine au niveau d'une position correspondant à la position 469 selon la SEQ ID NO:13, ou son complément ; une thymine au niveau d'une position correspondant à la position 553 selon la SEQ ID NO: 14, ou son complément ; ou une thymine au niveau d'une position correspondant à la position 567 selon la SEQ ID NO:22, ou son complément ;
l'agent thérapeutique comprenant un antioxydant, un agent de blocage du canal calcique, un médicament anti-inflammatoire, un stéroïde, un inhibiteur d'apoptose, de la D-méthionine, de l'ébsélène, de la N-acétylcystéine, de l'acide lipoïque, la combinaison d'ébsélène et d'allopurinol, du resvératrol, un facteur neurotrophe, du T-817MA, un inhibiteur de caspase, du z-DEVD-fmk, un inhibiteur de transport de cuivre, de la cimétidine et du sulfate de cuivre, ou un micronitrument doté d'une vitamine antioxydante.
